# FASCICULE DE BREVET EUROPEEN

(11) **EP 1 487 768 B1**
(45) Date de publication et mention de la délivrance du brevet: **19.09.2007**
(21) Numéro de dépôt: 03725271.5
(22) Date de dépôt: 06.03.2003
(51) Int. Cl.: C07C 11/06, C07C 2/12

(54) **PROCEDE DE CONVERSION EN PLUSIEURS ETAPES D'UNE CHARGE COMPRENANT DES OLEFINES A QUATRE, CINQ ATOMES DE CARBONE ET PLUS, EN VUE DE PRODUIRE DU PROPYLENE**
MEHRSTUFENVERFAHREN ZUR UMSETZUNG EINER BESCHICKUNG, DIE OLEFINE MIT DREI, VIER ODER MEHR KOHLENSTOFFATOMEN ENTHÄLT, ZUR HERSTELLUNG VON PROPYLEN
MULTI-STEP METHOD OF CONVERTING A CHARGE CONTAINING OLEFINS WITH FOUR, FIVE OR MORE CARBON ATOMS IN ORDER TO PRODUCE PROPYLENE

(30) Priorité: 15.03.2002 FR 0203211
(43) Date de publication de la demande: 22.12.2004
(73) Titulaire: Institut Français du Pétrole, 92852 Rueil-Malmaison Cedex (FR)
(72) Inventeur: DUPLAN Jean-Luc, 69540 Irigny (FR); BAYLE, Jérome, 44700 Orvault (FR); LACOMBE, Sylvie, 69230 St Genis Laval (FR); THOMAZEAU, Cécile, F-69530 Brignais (FR)
(86) Numéro de dépôt international: PCT/FR2003/000728
(87) Numéro de publication internationale: WO 2003/078364

(56) Documents cités:
- EP-A- 0 109 059
- WO-A-94/12452
- WO-A-99/29805
- FR-A- 2 755 958

## Description

L'invention concerne un procédé de production de propylène à partir de fractions légères d'hydrocarbures contenant notamment des butènes et/ou des pentènes.

Elle concerne plus particulièrement un procédé permettant de convertir au moins en partie en propylène une charge oléfinique, c'est-à-dire comprenant des oléfines, d'hydrocarbures dont le nombre de carbone est supérieur ou égal à 4, par exemple une coupe C4 et/ou C5 (le terme Cn indiquant une coupe d'hydrocarbures à n atomes de carbone), par exemple de vapocraquage ou de FCC. Le terme FCC, abréviation de « Fluid Catalytic Cracking » dans la terminologie anglo-saxonne, signifie craquage catalytique (en lit) fluidisé. De façon générale, et selon la présente invention, le terme FCC désigne le procédé classique utilisé en raffinerie, de craquage catalytique de fractions pétrolières lourdes, utilisant une charge bouillant principalement au dessus de environ 350 °C (au moins 50 % poids, généralement au moins 70 % poids et souvent 100 % poids de la charge bouillant au dessus de 350°C), par exemple du distillat sous vide, ou éventuellement du résidu atmosphérique.

Ces coupes C4/C5 oléfiniques sont disponibles en quantité importante, souvent excédentaire, dans les raffineries de pétrole et les installations de vapocraquage. Or leur recyclage est problématique :
- leur recyclage au vapocraquage présente des inconvénients (les rendements en oléfines légères sont moins élevés qu' avec des coupes paraffiniques et leur tendance à la formation de coke est élevée),
- leur recyclage au FCC n'est par ailleurs guère envisageable car elles sont très peu réactives dans les conditions du FCC, adaptées à la charge de distillat sous vide. Leur recyclage au FCC nécessiterait donc l'utilisation de conditions plus sévères ou de catalyseurs spécifiques, ce qui modifierait le fonctionnement du FCC.

La charge du procédé selon l'invention peut également comprendre une essence de vapocraquage ou une essence de FCC, ou une autre essence oléfinique. (On entend généralement par essence une coupe d'hydrocarbures issue pour sa plus grande partie au moins d'au moins une unité de conversion ou de synthèse (telle que FCC, viscoréduction, cokéfaction, unité Fischer-Tropsch, etc...) et dont la plus grande partie et typiquement au moins 90 % poids de cette coupe est constituée par des hydrocarbures ayant au moins 5 atomes de carbone et un point d'ébullition inférieur ou égal à environ 220°C).

La coupe oléfinique constituant la charge du procédé est donc de préférence choisie parmi celles définies précédemment, ou comprend un mélange de celles définies précédemment. Une charge typique comprend souvent des butènes et/ou des pentènes en quantité notable ou importante, mais peut comprendre également de l'éthylène, éventuellement de petites quantités de propylène non fractionné, des hexènes, des oléfines ayant de 7 à 10 atomes de carbone, des coupes d'essence oléfinique. Le plus souvent, la charge n'est pas purement oléfinique mais comprend aussi des paraffines (notamment du n-butane et/ou de l'iso-butane, des pentanes, et parfois des aromatiques notamment du benzène et/ou du toluène, et/ou des xylènes. Elle peut comprendre de l'isobutène et/ou des iso-amylènes.

La charge comprend également souvent des composés fortement insaturés : diènes (dioléfines) à 4, 5 atomes de carbones notamment (en particulier du butadiène).

La charge est typiquement une charge légère, dont le point final de distillation (selon la méthode TBP, bien connue de l'Homme du métier), ou au moins le point ou 90 % poids de la charge est distillée, est très généralement inférieur à 320°C, et généralement inférieur à 250°C.

Le procédé de conversion d'une charge comprenant des hydrocarbures oléfiniques en C4 et/ou C5, en une coupe comprenant du propylène, objet de la présente invention, met en oeuvre successivement l'enchaînement des étapes suivantes :
- une étape d'oligomérisation et/ou de co-oligomérisation des butènes et/ou pentènes contenus dans la charge, pour obtenir notamment des oléfines supérieures, notamment de nombre d'atomes de carbone majoritairement supérieur ou égal à huit. Si la charge, selon une variante du procédé selon l'invention, comprend de l'éthylène, les réactions de co-oligomérisation peuvent aussi produire une certaine quantité d'oléfines en C6 ou C7,
- une étape de craquage catalytique des oléfines supérieures ainsi produites.
Souvent, on appelle oligomères d'oléfines, des composés formés par l'addition de n oléfines identiques, et l'on appelle co-oligomères des composés formés par l'addition de n oléfines dont deux au moins sont différentes.

Selon l'invention, et dans la suite de la présente description ainsi que dans les revendications, on utilisera le terme oligomères (et les termes oligomériser et oligomérisation) de façon plus large, en l'appliquant à des oléfines supérieures formées par addition de n oléfines identiques et/ou différentes (le terme s'appliquant donc aussi à une coupe comprenant des co-oligomères).

L'oligomérisation se distingue de la polymérisation par une addition de molécules en nombre limité, le chiffre n cité précédemment étant, pour la plus grande partie pondérale au moins des oligomères, compris entre 2 et 10, bornes comprises, et généralement entre 2 et 5, notamment entre 2 et 4. Les oligomères peuvent cependant comprendre des traces d'oléfines ayant été oligomérisées avec n > 10. Généralement ces traces représentent moins de 5 % poids par rapport aux oligomères formés.

L'installation pour mettre en oeuvre le procédé selon l'invention est de préférence installée à proximité ou sur un site de raffinage (raffinerie de pétrole), ou de pétrochimie (généralement vapocraqueur).

### Art antérieur :

Un procédé connu de production de propylène, en dehors des procédés de production conventionnels, que sont le FCC et le vapocraquage (ou le propylène est coproduit avec d'autres produits tels que notamment de l'essence ou de l'éthylène) est le procédé de métathèse qui convertit en propylène un mélange éthylène + n-butène. Ce procédé est décrit dans le brevet français FR 2 608 595.

Un des avantages du procédé selon l'invention par rapport à la métathèse, est de produire du propylène à partir de l'ensemble des composés oléfiniques des coupes C₄, C5 et éventuellement de coupes d'hydrocarbures de nombre d'atomes de carbone supérieur, notamment d'essence, et de ne pas requérir la consommation massive d'éthylène, produit de coût élevé. S'il est appliqué sur un site de vapocraquage, le procédé selon l'invention permet non seulement de ne pas utiliser d'éthylène comme charge, mais également de coproduire de l'éthylène avec le propylène. La coproduction d'éthylène étant typiquement inférieure à celle de propylène, ceci permet d'améliorer le ratio propylène sur éthylène du vapocraqueur.

Par ailleurs, s'il est appliqué en raffinerie de pétrole, le procédé selon l'invention permet au contraire de valoriser au besoin (en sus de coupes C4/C5) des quantités relativement limitées et/ou difficilement valorisables d'éthylène, ce qui est souvent le cas en raffinerie.

On connaît également des procédés de production de propylène à partir de coupes C4 et C5 oléfiniques en une seule étape :
- on connaît notamment un procédé qui consiste essentiellement en un craquage catalytique en lit fluidisé, de technologie voisine du FCC classique, mais travaillant dans des conditions de hautes températures et sévérités, notamment : température de sortie du « riser » voisine de 700°C (le terme anglo-saxon "riser" signifiant élévateur tubulaire vertical à circulation ascendante de catalyseur et de charge réactionnelle). Un inconvénient de ce type de procédé, parfois appelé FCC pétrochimique, est de réaliser un surcraquage de l'essence comprise dans la charge qui diminue donc le rendement de cette dernière. Les paraffines contenues dans la charge subissent également ces conditions sévères de température, ce qui peut induire un craquage thermique et la formation de composés légers peu valorisables par ce procédé, par exemple des composés plus légers que le propylène. Par ailleurs, le rendement annoncé en propylène ne dépasse guère 30 %, même dans des conditions de très haute sévérité.
- Un autre procédé de production de propylène est un procédé en lit fluidisé utilisant comme catalyseur une zéolithe ZSM-5. Ce procédé est décrit dans la demande internationale WO 01/04237 ainsi que dans l'article « Maximizing Refinery Propylene Production Using ZSM-5 Technology », que l'on peut traduire par "Maximiser la production de propylène en raffinerie grâce à technologie ZSM-5" paru dans la revue "Hart's Fuel Technology and Management" ([revue Hart de] technologie et de gestion des combustibles), numéro de mai 1998. Les conditions opératoires typiques de ce procédé sont une température au voisinage de 600°C, une pression de 0,1 à 0,2 MPa.

Dans ces conditions, le rendement en propylène est d'environ 30 % et peut augmenter jusqu'à 50 % avec le recyclage des coupes C4 et C5 qui n'ont pas réagi.
Un inconvénient de ce procédé est qu'il est assez exigeant sur le plan des conditions opératoires, sévères, et de la définition des coupes d'entrée, C4 et C5 oléfiniques, qui ne peuvent pas être des essences à nombre d'atomes de carbone supérieur à 5, comme dans le cas de la présente invention qui peut traiter une coupe essence. Un autre inconvénient est que les paraffines de la charge, qui traversent le réacteur sans être converties catalytiquement, peuvent être partiellement craquées, notamment thermiquement, avec formation indésirable de composés légers.

Dans la famille des procédés en une étape, on peut également citer un procédé dont on trouvera une description dans l'article « Production Propylene from Low Valued Olefins » (Production de propylène à partir d'oléfines de faible valeur), paru dans la revue "Hydrocarbon Engineering" (Ingéniérie des hydrocarbures) de mai 1999. Il s'agit d'un procédé en lit fixe dont le catalyseur est une zéolithe de type ZSM-5 agissant en présence de vapeur d'eau. La température est voisine de 500°C et la pression est comprise entre 0,1 et 0,2 MPa. La durée de cycle annoncée est de l'ordre de 1000 heures. Le catalyseur est régénéré in situ et sa durée de vie globale, c'est à dire la période pendant laquelle il est utilisé dans le réacteur avant son renouvellement complet, est d'environ 15 mois. Le rendement en propylène annoncé est de 40 % environ et pourrait monter à 60 % avec le recyclage des coupes C4 et C5 qui n'ont pas réagi. Ce procédé permet d'obtenir un rendement en propylène relativement élevé. Il requiert cependant l'utilisation de vapeur d'eau, et la durée de cycle du catalyseur n'est pas très élevée.

On peut également citer un procédé décrit dans la demande internationale WO 99/29805 et dans le brevet EP-A- 1 061 116. Il s'agit d'un procédé utilisant un catalyseur zéolithique de type MFI ayant un rapport Si/Al élevé (de 180 à 1000) pour limiter les réactions de transfert d'hydrogène responsables de la production de diènes et d'aromatiques. La température est voisine de 550°C, la pression voisine de 0,1 MPa, et la vitesse spatiale comprise entre 10 h-1 et 30 h-1. Le rendement en propylène est compris entre 30 et 50 %, rapporté à la quantité d'oléfines contenues dans la charge. On peut donc l'estimer à typiquement moins de 30 % ramené directement à la charge entrante.

On peut également citer un procédé décrit dans le brevet EP-B1-0 109 059. Il s'agit d'un procédé utilisant un catalyseur zéolithique de type ZSM-5 ou ZSM-11 ayant des caractéristiques particulières, utilisé avec une vitesse spatiale élevée. Ce procédé décrit également, dans une variante, l'enchaînement de l'oligomérisation d'une coupe C4, l'élimination des butanes n'ayant pas réagi, et le craquage catalytique des oligomères. Le but de l'oligomérisation est l'élimination des butanes avant l'étape de craquage, via une oligomérisation des butènes rendant aisé le fractionnement butanes/oligomères alors que le fractionnement butanes/butènes est difficile. Les coupes C5 oléfiniques sont craquées directement. Il n'est pas mentionné de moyens techniques ou dispositions de procédé particulières lorsque la charge comprend de l'isobutène et/ou de l'isopentène, et/ou des isoamylènes.

Enfin, on peut également citer le brevet US 6,049,017, qui décrit un procédé de production de propylène et d'éthylène à partir de coupes oléfiniques en C4 comportant une étape d'élimination de l'isobutène par éthérification. Dans le procédé selon la présente invention, l'élimination de l'isobutène, même si elle est possible ou préférable, n'est pas indispensable, comme il sera expliqué plus loin.

### Description détaillée de l'invention :

De manière générale, par rapport aux procédés de conversion en une étape qui utilisent un seul catalyseur et un seul ensemble de conditions opératoires, le procédé selon l'invention, qui fait appel à deux étapes distinctes, permet d'accroître la longueur de chaîne des oléfines avant de les envoyer à l'étape de craquage afin de les rendre plus réactives à ce craquage, et d'optimiser chacune des étapes du point de vue du catalyseur et des conditions opératoires, comme il sera développé plus loin.
Les conditions opératoires différentes permettent notamment dans l'étape d'oligomérisation de favoriser les réactions d'addition, notamment par l'utilisation d'une pression relativement élevée, alors que l'on utilise de préférence une pression relativement basse et une température plus élevée pour l'étape de craquage. Ainsi, notamment, le propylène formé à l'étape de craquage a très peu tendance à s'oligomériser une fois formé.

Il a également été trouvé qu'une oligomérisation préalable d'une charge comprenant à la fois des oléfines en C4 et au moins une quantité notable d'autres oléfines du groupe constitué par les oléfines en C2, C5 et C6, notamment en C5 et/ou C6 conduisait à des rendements accrus et à une meilleure sélectivité en propylène.
Notamment, une oligomérisation (avec probablement co-oligomérisation partielle) d'un mélange comprenant des oléfines en C4 et en C5, ou bien en C4 et en C5 et en C6, ou bien en C4 et en C2 et en C5 conduit à des rendements en propylène (après craquage) améliorés, à une conversion supérieure, et à des conditions opératoires plus faciles à mettre en oeuvre, que si l'on n'oligomérisait que la coupe C4, les oléfines en C5 notamment étant craquées sans oligomérisation préalable. L'avantage de cette co-oligomérisation est notable lorsque la quantité de coupe C5 oligomérisée est suffisante.
Parmi les charges préférées du procédé selon l'invention, qui sont alimentées à l'étape b) d'oligomérisation, on trouve des charges comprenant au moins 50 % poids et souvent au moins 70 % poids voire plus de fractions en C4 + C5 + C6, et qui comprennent des oléfines d'au moins deux des fractions C4, C5, et C6, et notamment une charge:
- comprenant une coupe C4 oléfinique (c'est à dire comprenant des oléfines, avec éventuellement d'autres composés, par exemple des paraffines), la charge comprenant par exemple au moins 10 % poids d'oléfines en C4, et comprenant également des oléfines en C5 et/ou en C6, par exemple au moins 10 % poids, avec un rapport massique:
   R1 = Oléfines en C5 + Oléfines en C6 / Oléfines en C4 qui est supérieur à 0,15 et par exemple 0,2 < R1 < 5 notamment 0,3 < R1 < 3 en particulier 0,5 < R1 < 2 et plus particulièrement 0,7 < R1 < 1,5 .
- ou comprenant une coupe C4 oléfinique, la charge comprenant par exemple au moins 10 % poids d'oléfines en C4, et comprenant également des oléfines en C5, par exemple au moins 10 % poids, avec un rapport massique:
   R2 = Oléfines en C5 / Oléfines en C4 supérieur à 0,15 et par exemple 0,2 < R2 < 5 notamment 0,3 < R2 < 3 en particulier 0,5 < R2 < 2 et plus particulièrement 0,7 < R2 < 1,5.

Ces charges contenant des oléfines en C4 et C5 peuvent comprendre également des oléfines en C6; elles peuvent également être sensiblement exemptes d'oléfines en C6, avec par exemple avec un rapport massique:
R3 = Oléfines en C4 + Oléfines en C5 / Oléfines en C6 supérieur à 10, les oléfines en C6 étant envoyées par exemple à l'étape de craquage, en mélange avec les oligomères, sans être soumises à une oligomérisation préalable.
Ces charges donnent de bons rendements en propylène, après oligomérisation et craquage selon le procédé de l'invention. On pense que les oligomères d'oléfines en C4 et C5, en particulier les fractions de co-dimères en C9, provenant de la dimérisation d'un butène et d'un pentène donnent de meilleurs rendements en propylène et un ratio propylène/éthylène plus élevé que par craquage direct d'oléfines en C4 ou en C5, en particulier du fait qu'une fraction notable du dimère en C9 peut craquer en donnant 3 molécules de propylène.
Les fractions issues du craquage catalytique des oligomères contiennent typiquement des quantités relativement faibles de coupes C4 et C5 oléfiniques. La partie principale de la charge est typiquement une fraction oléfinique externe, de charge fraiche, c'est à dire ne provenant pas des effluents de l'étape d) du procédé selon l'invention, par exemple une ou plusieurs charges provenant des effluents d'un vapocraqueur (craquant par exemple du naphta), et/ou d'un FCC craquant principalement du distillat sous vide. Ceci permet notamment de pouvoir oligomériser une charge comprenant une quantité suffisante d'oléfines en C5, conforme aux valeurs précitées des rapports R1 et R2.

Il résulte du procédé selon l'invention divers avantages:
- Une plus grande flexibilité dans le choix des charges: non seulement des coupes oléfiniques en C4 mais également avec des fractions oléfiniques: en C5 et/ou C6, voire C7, et éventuellement de l'éthylène, pouvant être alimentées à l'oligomérisation et/ou de l'essence oléfinique relativement lourde pouvant facilement être introduite à l'étape de craquage.
- Un rendement en propylène plus élevé.
- Un ratio propylène/éthylène plus élevé.
- Une conversion plus importante à l'étape de craquage, du fait de la réactivité accrue des oligomères.
- Une durée de cycle des catalyseurs de craquage (et également d'oligomérisation) plus importante, le craquage pouvant être réalisé dans des conditions plus douces, notamment avec une température moins élevée. Cette durée de cycle accrue permet de pouvoir utiliser sans problèmes opératoires un, ou des réacteurs de craquage catalytique en lit fixe ou mobile, et de pouvoir éviter l'utilisation de réacteurs en lit fluidisé, plus onéreux.

L'invention propose donc un procédé de conversion catalytique d'une charge hydrocarbonée comprenant des oléfines à 4 et/ou 5 atomes de carbone, ce procédé étant caractérisé par l'enchaînement des étapes suivantes :
- au moins une étape b) ou b1) ou b3) d'oligomérisation (les étapes b), b1) et b3) seront explicitées ultérieurement), dans laquelle on réalise dans au moins un réacteur une oligomérisation catalytique des oléfines à 4 et/ou 5 atomes de carbone contenues dans la charge, en oléfines supérieures, c'est-à-dire en oligomères ayant un nombre d'atomes de carbone majoritairement supérieur ou égal à huit, puis,
- une étape d) dans laquelle on réalise un craquage catalytique d'une partie au moins, généralement une partie substantielle (telle que au moins 20 % poids ou au moins 30 % poids), et souvent la plus grande partie (plus de 50 % poids, souvent plus de 70 %, ou même 100 %) au moins des oligomères produits, dans un réacteur distinct du réacteur d'oligomérisation, pour produire notamment du propylène.

La charge avant d'être introduite dans l'unité pourra de préférence subir préalablement une hydrogénation sélective, dans une étape a) afin d'éliminer les dioléfines et autres impuretés acétyléniques souvent présentes dans la charge. On a trouvé en effet que ces différents composés fortement insaturés contribuent à une certaine désactivation du catalyseur d'oligomérisation et que l'hydrogénation sélective permet d'augmenter la quantité d'oléfines convertibles.

Selon une variante du procédé selon l'invention, l'effluent de l'étape b) d'oligomérisation est soumis à une étape c) de fractionnement comprenant une distillation pour séparer une partie au moins des composés à 4 et/ou 5 atomes de carbone, qui est souvent évacuée directement sans alimenter le réacteur de craquage catalytique. La fraction C4/C5 n'ayant pas réagi à l'oligomérisation est en effet essentiellement paraffinique et très peu réactive au craquage catalytique. Son évacuation directe évite un transit dans le réacteur de craquage catalytique pouvant générer par un certain craquage thermique la production indésirable de gaz. On peut envoyer cette charge C4/C5 dans une unité de vapocraquage cette charge paraffinique se révèlant être une bonne charge de vapocraquage.
On peut également évacuer la fraction C6 et ou la fraction C7 de l'effluent de l'étape b) d'oligomérisation, et l'envoyer éventuellement au vapocraquage, ainsi que la fraction C3- des composés à 3 atomes de carbone ou moins de ces mêmes effluents d'oligomérisation.

L'effluent de l'étape d) de craquage catalytique est typiquement soumis à une étape e) de fractionnement comprenant le plus souvent une compression des gaz et une ou plusieurs distillations pour séparer les effluents et produire une coupe C3 riche en propylène, ou du propylène sensiblement pur.

Une partie des composés à 4 et/ou 5 atomes de carbone contenus dans les effluents de craquage peut avantageusement être recyclée à l'entrée de l'étape b) ou de l'étape a).
On décrit ci-après plus en détail les conditions particulières des différentes étapes réactionnelles du procédé selon l'invention, selon une variante comprenant une hydrogénation sélective, une oligomérisation et un craquage catalytique intégrés sur un même site, la charge utilisée étant une coupe légère d'hydrocarbures en C4 et C5 contenant principalement des butènes, des pentènes, des butanes, des pentanes ainsi que dans certains cas, du butadiène et du pentadiène en quantité variable.

### 1) Hydrogénation sélective (étape a)) :

La coupe légère provient typiquement d'un craqueur catalytique et/ou d'un vapocraqueur. Les teneurs en diènes et en acétyléniques sont importantes quand cette coupe vient d'un vapocraqueur ; c'est pourquoi l'étape d'hydrogénation sélective des diènes et des acétyléniques en oléfines est quasiment indispensable dans ce cas. Elle est également préférable dans la plupart des cas, car elle réduit le cokage du catalyseur d'oligomérisation à l'étape b), et accroît la durée de cycle du réacteur d'oligomérisation. On ne sortirait toutefois pas du cadre de l'invention si une telle étape d'hydrogénation sélective n'est pas comprise dans le procédé selon l'invention.

L'objet principal de cette première étape est de transformer les dioléfines (ou diènes) en mono-oléfines. En effet, les mono-oléfines sont la source des oligomères produits dans l'étape 2. Il est donc souhaitable de transformer les dioléfines en mono-oléfines. Le deuxième objet de cette étape est d'éliminer les traces d'hydrocarbures acétyléniques toujours présentes dans ces coupes et qui sont des composés indésirables pour l'oligomérisation, ces composés étant également transformés en mono-oléfines.

Lorsque la proportion de dioléfines dans la coupe est importante, on peut avantageusement effectuer la transformation dans deux, ou trois réacteurs en série afin de mieux contrôler la sélectivité de l'hydrogénation. Souvent on dilue la charge à traiter par recyclage d'un certain débit de l'effluent de cette hydrogénation sélective.

La teneur résiduelle en dioléfines + acétyléniques de l'effluent de l'hydrogénation sélective est typiquement inférieure à environ 1000 ppm poids, de préférence inférieure à environ 100 ppm poids et de façon très préférée inférieure à 20 ppm poids. La teneur résiduelle en acétyléniques peut même être inférieure à 10 ppm, ou 5 ppm, ou même à 1 ppm poids.

La quantité d'hydrogène nécessaire à l'ensemble des réactions réalisées dans cette étape est généralement ajustée en fonction de la composition de la coupe pour avoir avantageusement seulement un léger excès d'hydrogène par rapport à la stoechiométrie.

Généralement, cette étape d'hydrogénation sélective est réalisée en utilisant un catalyseur comprenant au moins un métal choisi dans le groupe formé par le nickel, le palladium, et le platine, déposé sur un support comprenant de l'alumine, de la silice ou de la silice-alumine. On met en oeuvre de préférence un catalyseur qui comprend au moins du palladium ou un composé de palladium fixé sur un support minéral réfractaire, par exemple sur une alumine ou une silice-alumine. La teneur en palladium sur le support peut être typiquement de 0,01 % à 5 % en poids, de préférence de 0,05 % à 1 % en poids. Divers modes de prétraitement connus de l'homme du métier peuvent éventuellement être appliqués à ces catalyseurs pour améliorer leur sélectivité d'hydrogénation vers les mono-oléfines.

La température opératoire de l'hydrogénation sélective est généralement comprise entre 0 et 200°C, la pression typiquement comprise entre 0,1 et 5 MPa, souvent entre 0,5 et 5 MPa, la vitesse spatiale typiquement comprise entre 0,5 et 20 m³ par heure et par m³ de catalyseur, souvent entre 0,5 et 5 m³ par heure et par m³ de catalyseur, et le rapport molaire H2/(composés acétyléniques + dioléfiniques) généralement compris entre 0,5 et 5 et de préférence entre 1 et 3.

Lorsque l'on alimente également au craquage catalytique une coupe essence, on peut également soumettre préalablement cette coupe à une hydrogénation sélective, commune ou séparée de celle de la coupe C4 et/ou C5. Lorsque cette hydrogénation sélective est commune, on peut éventuellement séparer l'essence de la coupe C4 et/ou C5 en amont de l'oligomérisation.

On utilise généralement, pour réaliser l'hydrogénation sélective un réacteur en lit fixe, traversé à co-courant descendant par la charge à traiter et de l'hydrogène, ou à courant descendant pour la charge à traiter et à courant ascendant pour de l'hydrogène.

Le procédé de l'invention peut également comprendre une ou plusieurs étapes éventuelles de purification de la charge (différente(s) ou commune(s) avec l'hydrogénation sélective) en amont de l'oligomérisation, qui peuvent être utiles ou nécessaires à l'une au moins des étapes suivantes: oligomérisation et craquage. L'utilité de ces étapes optionnelles de purification dépend directement du ou des catalyseurs employés ainsi que des conditions opératoires et apparaîtra clairement à l'homme du métier pour chaque cas particulier considéré. Ainsi on ne sortirait pas du cadre de l'invention si l'on réalisait en amont de l'oligomérisation une ou plusieurs étapes de désulfuration, et/ou de séchage, et/ou de déazotation, et/ou de déoxygénation, pour éliminer l'une ou plusieurs des impuretés suivantes: soufre, eau, azote, oxygène, en dessous de 100 ppm, ou 10 ppm, ou même 1 ppm poids, selon des techniques conventionnelles.

### 2) Oligomérisation (étape b)) :

L'objet de la deuxième étape est d'oligomériser les oléfines linéaires, et éventuellement branchées en C₄ et C₅, ainsi que d'autres oléfines éventuellement présentes, par exemple et de façon non limitative des oléfines en C2 (éthylène) et/ou en C6 (hexènes), issues de l'étape précédente, pour obtenir un mélange d'hydrocarbures contenant des mono-oléfines avec un nombre d'atomes de carbone majoritairement supérieur ou égal à huit. Typiquement, à partir d'une charge en C4, on obtient des oligomères dont le nombre d'atomes de carbone est en grande partie au moins inférieur ou égal à 30, et pour la plus grande partie compris entre 8 et 20.

L'oligomérisation peut être réalisée en une ou plusieurs étapes, avec un ou plusieurs réacteurs et un ou plusieurs catalyseurs. La description qui suit du catalyseur et des conditions opératoires peut s'appliquer à l'une quelconque des étapes et/ou à l'un quelconque des réacteurs. -

L'étape d'oligomérisation peut utiliser un catalyseur comprenant un acide de Lewis, par exemple du chlorure d'aluminium, un chloroalkylaluminium, du tétrachlorure d'étain, du trifluorure de bore, cet acide de Lewis étant souvent associé à des traces d'acide chlorhydrique, d'eau, de chlorure de butyle tertiaire, ou d'acides organiques.
Les sélectivités en dimère et en trimère dépendent du catalyseur et des conditions opératoires. Dans la présente invention, le procédé d'oligomérisation est tel qu'on cherche une transformation notable ou éventuellement poussée de l'ensemble des oléfines de départ.

Le catalyseur utilisé pour l'étape d'oligomérisation peut comprendre également de l'acide sulfurique supporté ou de l'acide phosphorique supporté, par exemple sur silice, alumine, ou silice-alumine.

Le catalyseur utilisé pour l'étape d'oligomérisation peut comprendre également une résine sulfonique (par exemple non limitatif, une résine AMBERLIST commercialisée par la société ROHM & HAAS).

Le catalyseur utilisé pour l'étape d'oligomérisation peut comprendre également une silice-alumine, ou de préférence un solide acide présentant une sélectivité de forme.

Par exemple ce catalyseur peut comprendre au moins une zéolithe présentant une sélectivité de forme, cette zéolithe comprenant du silicium et au moins un élément choisi dans le groupe constitué par l'aluminium, le fer, le gallium, le phosphore, le bore, et de préférence l'aluminium. Cette zéolithe peut être par exemple de l'un des types structuraux suivants : MEL (par exemple la ZSM-11), MFI (par exemple la ZSM-5), NES, EUO, FER, CHA (par exemple la SAPO-34), MFS, MWW, ou peut également être l'une des zéolithes suivantes : NU-85, NU-86, NU-88 et IM-5, qui présentent également une sélectivité de forme.

L'avantage de ces zéolithes présentant une sélectivité de forme est qu'il limite la formation d'oligomères fortement branchés par exemple d'isomères tri-branchés dont le craquage conduit à une sélectivité moindre propylène / isobutène, c'est à dire à un rapport massique propylène / isobutène moins élevé.

On peut également utiliser plusieurs zéolithes présentant une sélectivité de forme, par exemple une zéolithe de type MFI (par exemple la ZSM-5) associée à une autre zéolithe, précédemment citée ou de l'un des types précédemment cités.

La zéolithe utilisée peut aussi être mélangée avec une zéolithe ne présentant pas de sélectivité de forme, telle que par exemple une zéolithe Y de type structural FAU.

La ou les zéolithes peuvent être dispersées dans une matrice à base de silice, d'alumine ou de silice alumine, la proportion de zéolithe (et généralement de zéolithe présentant une sélectivité de forme) étant souvent comprise entre 3 et 80 % poids, notamment entre 6 et 50 % poids et préférentiellement entre 10 et 45 % poids.

La zéolithe utilisée (ou les zéolithes utilisées) présentant une sélectivité de forme présente(nt) généralement un rapport Si/Al supérieur à 12, préférentiellement supérieur à 40, de manière encore préférée supérieur à 50, et de manière encore davantage préférée supérieur à 80.

Le rapport Si/Al peut par exemple être compris entre 40 et 1000. Ceci permet de réduire l'acidité du catalyseur et les réactions de transfert d'hydrogène qui conduisent à la formation de paraffines non ou peu réactives au niveau de l'étape ultérieure de craquage. De tels rapports Si/Al élevés peuvent être obtenus au moment de la fabrication de la zéolithe, ou par désalumination ultérieure.

Le catalyseur d'oligomérisation peut enfin être différent des catalyseurs précédemment cités, s'il possède une activité notable en oligomérisation.

Le catalyseur peut être mis en oeuvre à l'état solide, en poudre, ou bien sous forme de billes ou d'extrudés de diamètre généralement compris entre 0,4 et 6 mm, de préférence entre 0,6 et 4 mm.

Le catalyseur peut également être mis en oeuvre sous forme d'une suspension dans un hydrocarbure saturé tel que l'hexane ou l'isobutane, ou dans un hydrocarbure halogéné tel que le chlorure de méthyle. La suspension peut être utilisée en lit bouillonnant, notamment avec des particules de diamètre moyen compris entre 0,25 et 1 mm et de préférence entre 0,3 et 0,8 mm, ou en suspension fine, avec des particules de diamètre moyen compris entre 0,02 et 0,25 mm et de préférence entre 0,03 et 0,20 mm. On peut également utiliser une suspension ou les particules sont à l'état colloïdal.
La mise en oeuvre préférée pour le réacteur d'oligomérisation est celle en lit fixe.

Les conditions opératoires sont choisies en fonction du catalyseur, de telle façon que la réaction se déroule à une vitesse suffisante. La température (de sortie réacteur) peut être par exemple comprise entre -100°C et 350°C, de préférence entre 0°C et 310 °C, et de façon très préférée entre 70°C et 310°C, par exemple entre 120°C et 250°C, notamment entre 150 et 220 °C. Souvent la température de l'étape b) d'oligomérisation est inférieure d'au moins 40°C, de préférence d'au moins 80°C, et de façon très préférée d'au moins 120°C à celle de l'étape d) de craquage catalytique.

La pression est typiquement comprise entre 0,1 et 10 MPa, et de préférence entre 0,1 et 5 MPa, et de façon très préférée entre 0,8 et 4 MPa, et notamment entre 1,5 et 3,5 MPa. Souvent la pression (en sortie du réacteur) de l'étape b) d'oligomérisation est supérieure d'au moins 0,5 MPa, de préférence d'au moins 1 MPa, et de façon très préférée d'au moins 1,5 MPa à celle de l'étape d) de craquage catalytique.

La VVH est généralement comprise entre 0,1 et 5 m³ par heure et par m³ de catalyseur, et de préférence entre 0,5 et 4 m³ par heure et par m³ de catalyseur.

Les conditions opératoires sont souvent également optimisées en fonction des caractéristiques de la charge.
On peut également utiliser pour l'étape a) d'hydrogénation sélective et pour l'étape b) d'oligomérisation des conditions voisines, et notamment des pressions voisines, telles que des pressions ne différant entre elles que de 0,5 MPa au plus, ou même 0,3 MPa au plus. Ceci permet d'enchaîner les deux réactions successivement, éventuellement sans fractionnement ni pressurisation ni dépressurisation intermédiaire, ou même éventuellement sans refroidissement ou même sans réchauffage intermédiaire. Il est également possible de réaliser les réactions d'hydrogénation sélective et d'oligomérisation dans deux lits successifs d'un même réacteur.

La conversion des oléfines en C4 et C5 au cours de l'oligomérisation atteint généralement 70 %, ou 90 % ou plus, et peut même être sensiblement totale.

Il peut être intéressant dans cette étape, dans des conditions particulières discutées ci-après, d'ajouter à la charge une petite quantité d'éthylène qui favorise la formation d'oligomères à six ou sept atomes de carbone (par addition avec des oléfines en C4/C5 de la charge) et leur craquage ultérieur en propylène. Ceci permet de valoriser les quantités relativement limitées d'éthylène disponibles dans une raffinerie de pétrole (cet éthylène étant essentiellement produit au FCC). Une autre situation ou cette disposition est intéressante est celle d'un appoint d'éthylène venant d'un vapocraqueur, dans une période conjoncturelle ou la demande en éthylène est faible alors que la demande en propylène reste élevée. On peut alors ajuster la quantité d'éthylène à l'excédent disponible. (A titre de comparaison, cet ajustement n'est pas possible dans le procédé de métathèse, ou l'on utilise autant de moles d'éthylène que de butène). La quantité d'éthylène utilisable est par exemple comprise entre 0,5 et 15 % poids de la charge d'oligomérisation. Typiquement, la charge du réacteur d'oligomérisation comprend de 0,5 à 15 % poids d'éthylène par rapport à la somme des oléfines en C4, C5, et C6.

L'utilisation d'une oligomérisation à relativement haute pression et basse température par rapport à celle du craquage catalytique permet d'optimiser séparément les deux types de réactions chimiques et d'utiliser des catalyseurs spécifiques. Elle permet également d'augmenter la durée de cycle et la durée de vie du catalyseur d'oligomérisation, qui est soumis à des conditions beaucoup moins sévères, notamment vis-à-vis du cokage.

Généralement, le réacteur d'oligomérisation est un lit fixe, utilise un catalyseur comprenant une silice-alumine ou de préférence au moins une zéolithe, et de façon très préférée une zéolithe présentant une sélectivité de forme (par exemple une zéolithe de type MFI), travaille à une température comprise entre 70°C et + 310°C, une pression comprise typiquement entre 0,1 et 5 MPa, et une vitesse spatiale comprise entre 0,1 et 5 m³ par heure et par m³ de catalyseur.

Selon une variante du procédé selon l'invention, utilisable notamment lorsque la charge contient de l'isobutène, en particulier en quantité substancielle ou élevée, on peut réaliser l'étape b) d'oligomérisation en 3 étapes:
- Une étape b1) d'oligomérisation limitée, permettant de réaliser une oligomérisation préférentielle des oléfines branchées, plus réactives, notamment de l'isobutène, les oléfines linéaires étant notablement moins oligomérisées,
- Une étape b2) de fractionnement des effluents de l'étape b1), par exemple par distillation ou tout autre fractionnement connu, permettant d'extraire au moins une coupe comprenant du di-isobutène et/ou, optionnellement, du tri-isobutène: coupe C8 riche en di-isobutène, ou di-isobutène sensiblement pur, ou éventuellement une coupe C8+ (C8 et plus lourds, pouvant éventuellement également comprendre du tri-isobutène en même temps que du disobutène), cette coupe extraite étant évacuée directement (c'est-à-dire n'étant pas alimentée aux étapes subséquentes b3) d'oligomérisation et d) de craquage).
- Une étape b3) d'oligomérisation finale de l'effluent de l'étape b2), ou au moins de fractions oléfiniques en C4 et/ou C5, après évacuation de la coupe précitée comprenant du di-isobutène et/ou optionnellement du tri-isobutène.
Ces étapes coopèrent entre elles ainsi qu'avec l'étape d) de craquage: Les étapes b1) et b2) permettent d'éliminer au moins en partie l'isobutène à travers un produit : le di-isobutène et/ou le tri-isobutène dont les rendements de craquage catalytique en propylène sont relativement faibles, et d'obtenir des oligomères moins branchés à l'étape b3), donnant de meilleurs rendements de craquage à l'étape d). L'élimination au moins partielle de l'isobutène avant l'étape b3) permet également de réduire la formation de gomme dans cette étape b3) ou l'on recherche une oligomérisation poussée des oléfines linéaires en C4 et/ou C5.
Le variante de procédé précédemment décrite, (avec oligomérisation limitée b1) puis oligomérisation finale b3) après fractionnement b2) et élimination au moins partielle des oligomères formés dans b1)), peut aussi être appliquée à une charge comprenant des isoamylènes (oléfines branchées en C5) à la place de l'isobutène, ou une charge comprenant de l'isobutène et des isoamylènes. Ces oléfines branchées peuvent être oligomérisées beaucoup plus facilement et préférentiellement à leurs homologues linéaires, ce qui permet de les évacuer au moins en partie après l'étape b1).
L'étape b1), qui ne vise pas à la formation d'oléfines linéaires bons précurseurs de propylène, peut être mise en oeuvre avec un catalyseur parmi ceux cités précédemment, mais également avec un catalyseur zéolithique ayant un pourcentage de zéolithe présentant une sélectivité de forme plus faible que celui de l'étape b3), ou même avec un catalyseur non zéolithique, comprenant essentiellement une silice-alumine amorphe d'acidité moyenne.
L'étape b1) d'oligomérisation peut aussi utiliser des conditions opératoires différent(e)s, très sélectif(ves) car isomérisant très préférentiellement ou exclusivement l'isobutène (et/ou les isoamylènes) par rapport aux n-butènes (butènes linéaires) et/ou aux n-pentènes. Par exemple on pourra utiliser des conditions plus douces en première étape d'oligomérisation par rapport à l'étape finale, notamment en utilisant une température plus basse d'au moins 40 °C en première étape. On peut par exemple réaliser une première oligomérisation b1) avec une température comprise entre 20 et 80 °C, et une seconde oligomérisation b3) avec une température supérieure à 100°C, voire 120 °C ou plus. On peut utiliser le même catalyseur pour b3), par exemple à base de silice-alumine, ou alternativement des catalyseurs différents.
Le di-isobutène et le tri-isobutène sont en fait, pour chacun de ces composés, un mélange d'isomères, connus de l'homme du métier; il y a notamment deux isomères pour le di-isobutène, dont le 2,4,4-triméthyl-2-pentène, de point d'ébullition normal 104,9°C qui bout dans le domaine de l'essence et a un bon indice d'octane. Le tri-isobutène comprend des oligomères dont certains ont un point d'ébullition normal compris entre 196 et 210 °C, qui peuvent être incorporés au moins en partie à une base d'essence ou à un kérosène, ou à un gazole, selon les valorisations recherchés. Il peut également être valorisé pour des usages en chimie.
Une coupe extraite riche en di-isobutène peut être valorisée à un niveau élevé comme base d'essence, ou pour d'autres applications, par exemple en chimie etc...

L'étape b1) peut notamment être mise en oeuvre sur une coupe C4 seule; une coupe C5, ou C2 et C5 peut alors éventuellement être ajoutée aux butènes non convertis dans b1) pour l'oligomérisation finale à l'étape b3). On peut également réaliser l'étape b1) avec une charge comprenant d'autres hydrocarbures qu'une coupe C4, par exemple une coupe coupe oléfinique en C4 et C5, ou C4 et C5 et C6, ou C4 et C2 ou C4 et C2 et C5, ou C4 et C2 et C5 et C6.
Lorsque l'oligomérisation est mise en oeuvre dans une étape b) unique, on peut également, de la même façon qu'après l'étape b1), prélever et évacuer directement une partie des oligomères produits, par exemple une fraction comprenant du di-isobutène et/ou du tri-isobutène.
Dans toutes ces variantes, on pourra aussi évacuer après une étape de fractionnement b2) ou c) des fractions peu réactives en C4 et/ou C5, pour ne pas encombrer les étapes subséquentes.

### 3) Craquage catalytique (étape d)) :

La charge alimentée à l'étape d) de craquage catalytique contient typiquement de 20 à 100 % poids, d'oléfines à au moins 8 atomes de carbone ayant été produites par oligomérisation d'oléfines légères à 4 et/ou 5 atomes de carbone, souvent de 30 à 100 % poids, et le plus souvent de 50 à 100 % poids, en particulier de 60 à 100 % poids.
La charge peut aussi comprendre d'autres oligomères formés essentiellement à partir du groupe constitué par les oléfines en C2 à C10, la quantité totale d'oligomères à au moins 6 atomes de carbone étant typiquement de 25 à 100 % poids, souvent de 35 à 100 % poids, le plus souvent de 55 à 100 % poids, en particulier de 65 à 100 % poids par rapport à la charge de l'étape d).
Les oligomères en C6, formés notamment par addition d'un butène avec de l'éthylène, ou les oligomères plus lourds formés au moins en partie à partir d'oléfines en C6 et plus (C6+) sont en effet également de bons précurseurs de propylène, qu'il est avantageux d'alimenter aussi au craquage catalytique.

Le catalyseur utilisé pour l'étape de craquage catalytique peut comprendre une silice-alumine. De préférence, cependant, on utilise un solide acide présentant une sélectivité de forme.

Par exemple, ce catalyseur peut comprendre au moins une zéolithe présentant une sélectivité de forme, cette zéolithe comprenant du silicium et au moins un élément choisi dans le groupe formé par l'aluminium, le fer, le gallium, le phosphore, le bore, et de préférence l'aluminium. Cette zéolithe présentant une sélectivité de forme peut être de l'un des types structuraux suivants : MEL (par exemple la ZSM-11), MFI (par exemple la ZSM-5), NES, EUO, FER, CHA (par exemple la SAPO-34), MFS, MWW, ou peut également être l'une des zéolithes suivantes: NU-85, NU-86, NU-88 et lM-5, qui présentent également une sélectivité de forme.

L'avantage de ces zéolithes présentant une sélectivité de forme est qu'il conduit à une meilleure sélectivité propylène / isobutène (rapport propylène / isobutène plus élevé dans les effluents de craquage) .

On peut également utiliser plusieurs zéolithes présentant une sélectivité de forme, par exemple une zéolithe de type MFI (par exemple la ZSM-5) associée à une autre zéolithe présentant une sélectivité de forme, précédemment citée ou de l'un des types précédemment cités.

La (ou les) zéolithe(s) présentant une sélectivité de forme, du groupe formé par les zéolithes de l'un des types structuraux suivants : MEL (par exemple la ZSM-11), MFI (par exemple la ZSM-5), NES, EUO, FER, CHA (par exemple la SAPO-34), MFS, MWW, ou du groupe des zéolithes suivantes: NU-85, NU-86, NU-88 et lM-5, peut également être mélangée avec une zéolithe ne présentant pas de sélectivité de forme, telle que par exemple une zéolithe Y de type structural FAU.
Souvent on utilise un catalyseur comprenant une ou plusieurs zéolithe(s) présentant une sélectivité de forme, la proportion de zéolithe(s) présentant une sélectivité de forme étant comprise entre 70 et 100 % poids, bornes comprises, par rapport à la quantité totale de zéolithe(s). On peut notamment utiliser un catalyseur dont la proportion de zéolithe(s) présentant une sélectivité de forme est comprise entre 80 et 100 % poids par rapport à la quantité totale de zéolithe(s), et même un catalyseur dont la ou les zéolithes présentent toutes une sélectivité de forme.

La ou les zéolithes peuvent être dispersées dans une matrice à base de silice, d'alumine ou de silice alumine, la proportion de zéolithe (et généralement de zéolithe présentant une sélectivité de forme) étant souvent comprise entre 3 et 80 % poids, de préférence entre 8 et 70 % poids, par exemple entre 15 et 60 % poids, notamment entre 20 et 50 % poids.
La zéolithe utilisée (ou les zéolithes utilisées) présentant une sélectivité de forme présente(nt) généralement un rapport Si/Al supérieur à 12, préférentiellement supérieur à 20, de manière encore préférée supérieur à 50, et souvent supérieur à 80. Il peut par exemple être compris entre 40 et 500. Ceci permet notamment de réduire l'acidité du catalyseur et les réactions de transfert d'hydrogène qui conduisent à la formation de paraffines au détriment de la formation de propylène.

De tels rapports Si/Al élevés peuvent être obtenus au moment de la fabrication de la zéolithe, ou par désalumination ultérieure.

Le catalyseur de craquage catalytique peut enfin être différent des catalyseurs précédemment cités, s'il possède une activité notable en craquage catalytique pour la production de propylène.

Les rapports Si/Al précités peuvent être différents pour les catalyseurs d'oligomérisation et de craquage, ce qui permet leur optimisation respective.

Le catalyseur peut être mis en oeuvre à l'état solide, en poudre si l'on utilise un réacteur en lit fluidisé, avec par exemple une dimension moyenne des particules comprise entre 0,02 et 0,5 mm, et de préférence entre 0,04 et 0,15 mm. Typiquement, le catalyseur circule alors de façon continue du réacteur de craquage vers une zone de régénération, puis retourne vers le réacteur. La technologie utilisée est alors voisine ou identique à celle du procédé FCC.

Les oligomères peuvent également être craqués selon le procédé FCC, en mélange avec du gasoil lourd et/ou du distillat sous vide (ou dans un "riser" séparé, le terme anglo-saxon riser signifiant élévateur). Cette variante fait l'objet d'une demande de brevet séparée, simultanée à la présente demande. Dans un tel cas, la quantité d'oligomères ajoutés à la charge lourde (gasoil lourd et/ou du distillat sous vide) est le plus souvent relativement faible: typiquement entre 3 et 40% poids, notamment de 4 à 30%, ou de 4 à 20% poids de la charge totale.
La charge traitée est typiquement une coupe oléfinique (C4 et/ou C5 et/ou C6 et/ou C2) issue du FCC. En général on ne traite qu' une quantité limitée de coupe oléfinique, qui conduit après craquage des oligomères dans le FCC (avec le distillat sous vide et/ou le gasoil lourd), à une augmentation de la quantité de gaz compatible avec le compresseur de gaz craqués et l'installation de traitement des gaz et des fractions légères C5/C6. On peut alors alimenter à l'oligomérisation (après traitements éventuels de purification) une quantité prédéterminée de coupe oléfinique (C4 et/ou C5 et/ou C6 et/ou C2), le complément par rapport à la production du FCC étant évacué et fractionné de façon à réaliser un courant de purge et éviter un gonflement excessif de la boucle de produits légers C5⁻ autour du FCC et de l'oligomérisation, notamment une accumulation d'isobutène et/ou d'isoamylènes. Ces composés ont en effet tendance à s'oligomériser rapidement mais à recraquer (lors de l'étape de craquage) en quantité notable ou totale vers le produit de départ, en ne produisant que très peu de propylène. Une purge permet d'éviter une accumulation croissante d'isobutène et/ou isoamylènes.

En alternative, il peut être mis en oeuvre en lit fixe ou en lit mobile, sous forme de billes ou d'extrudés de diamètre généralement compris entre 0,4 et 6 mm, de préférence entre 0,6 et 4 mm.

Selon l'une des variantes de réalisation préférées du procédé selon l'invention, on utilise pour l'étape d) de craquage un lit mobile de catalyseur, par exemple de billes de 1 à 3 mm de diamètre. Le catalyseur circule alors de façon continue ou semi-continue du réacteur de craquage vers une zone de régénération, puis retourne vers le réacteur.

Selon une autre variante de réalisation préférée, on utilise au moins 2 réacteurs en lit fixe avec un fonctionnement cyclique, un réacteur étant en fonctionnement (phase craquage) et un autre réacteur en phase de régénération, selon la technique des réacteurs en "swing" selon le terme anglo-saxon bien connu de l'homme du métier, qui signifie basculement. Lorsque la régénération du second réacteur est finie, on bascule la charge vers le second réacteur, et l'on régénère le catalyseur du premier réacteur. On peut également utiliser trois réacteurs dont deux réacteurs en fonctionnement et un en régénération, ou trois réacteurs en fonctionnement et un en régénération, ou N réacteurs en fonctionnement et P réacteurs en régénération, variantes considérées selon l'invention comme équivalents techniques assimilés à des réacteurs en swing.

La phase de régénération comprend typiquement une phase de combustion des dépôts carbonés formés sur le catalyseur, par exemple à l'aide d'un mélange air/azote ou d'air appauvri en oxygène (par exemple par recirculation de fumées), ou d'air, et peut éventuellement comprendre d'autres phases de traitement et de régénération du catalyseur.

On opère habituellement le craquage catalytique à une température d'environ 450 à environ 650°C et de préférence entre 480°C et 600°C avec un temps de séjour dans le réacteur inférieur à 1 minute, souvent d'environ 0,1 à environ 50 secondes et de préférence de 0,4 à 15 secondes. La pression opératoire est généralement comprise entre 0,1 et 5 MPa, le plus souvent comprise entre 0,1 et 1,5 MPa, et de préférence entre 0,1 et 0,5 MPa.

Les conditions de régénération du catalyseur de craquage utilisent généralement une température comprise entre 300 et 900°C, notamment entre 500 et 750°C, la pression étant le plus souvent voisine de la pression de craquage, ou bien proche de la pression atmosphérique.

Selon une autre variante de réalisation du procédé selon l'invention, on peut aussi utiliser un même catalyseur en circulation pour l'oligomérisation et le craquage.

Cette circulation du catalyseur peut être mise en oeuvre en lit mobile ou en lit fluidisé. Le catalyseur circule alors avantageusement entre trois zones : la zone d'oligomérisation, la zone de craquage catalytique, et une troisième zone de régénération du catalyseur, cette dernière zone réalisant notamment une élimination du coke déposé sur le catalyseur (par combustion contrôlée selon l'une des techniques connues de l'homme du métier). Par exemple, le flux de catalyseur régénéré issu de la zone de régénération peut être subdivisé en deux flux, le premier alimentant la zone d'oligomérisation, et le second la zone de craquage. Le catalyseur issu des deux zones réactionnelles (après séparation préalable des effluents réactionnels), peut alors être régénéré dans la zone commune de régénération.

Le flux de catalyseur peut également alimenter en série les deux zones réactionnelles (oligomérisation puis craquage, ou l'inverse).

Les flux de catalyseur peuvent éventuellement subir un refroidissement, notamment celui alimentant la zone d'oligomérisation, ou un refroidissement différent, pour obtenir des températures opératoires différentes dans la zone d'oligomérisation et dans celle de craquage. Ce refroidissement peut être obtenu par exemple par mise en contact du catalyseur avec un gaz plus froid, ou dans un échangeur de chaleur. Les charges réactionnelles peuvent également être alimentées à des températures différentes pour obtenir ce résultat. On peut également opérer le réacteur de craquage avec du catalyseur régénéré, relativement plus chaud.

Les variantes principales de mise en oeuvre du procédé selon l'invention sont les suivantes :
- Variante A : catalyseurs d'oligomérisation et de craquage différents ; oligomérisation en lit fixe, de préférence avec régénération cyclique du catalyseur dans le même réacteur à intervalles de temps éloignés, ou avec des réacteurs en swing ; craquage catalytique en lit fixe, de préférence avec régénération cyclique relativement fréquente avec autre(s) réacteur(s) en swing.
- Variante B : catalyseurs d'oligomérisation et de craquage différents ; oligomérisation en lit fixe, de préférence avec régénération cyclique du catalyseur dans le même réacteur à intervalles de temps éloignés, ou avec des réacteurs en swing; craquage catalytique en lit mobile (avec circulation continue ou semi-continue du catalyseur vers une zone de régénération).
- Variante C : catalyseurs d'oligomérisation et de craquage différents ; oligomérisation en lit fixe, de préférence avec régénération cyclique du catalyseur dans le même réacteur à intervalles de temps éloignés, ou avec des réacteurs en swing; craquage catalytique en lit fluidisé (avec circulation continue du catalyseur vers une zone de régénération).
- Variante D : catalyseur commun d'oligomérisation et de craquage ; oligomérisation et craquage en lit mobile (le catalyseur commun circulant dé façon continue ou semi-continue entre une zone d'oligomérisation, une zone de craquage, et une zone de régénération).
- Variante E : catalyseur commun d'oligomérisation et de craquage ; oligomérisation et craquage en lit fluidisé (le catalyseur commun circulant en continu entre une zone d'oligomérisation, une zone de craquage, et une zone de régénération).

Les variantes A, B, et C peuvent également être mises en oeuvre avec un catalyseur en suspension dans un liquide pour l'étape d'oligomérisation.

Les variantes préférées selon l'invention sont les variantes A, B, et C, notamment avec un lit fixe pour l'oligomérisation, et les variantes les plus préférées sont les variantes A et B.

Généralement, le rendement en propylène rapporté à la quantité d'oléfines contenues dans la charge fraîche du procédé est compris entre 30 % et 60 % poids, et souvent entre 40 et 60 % poids.

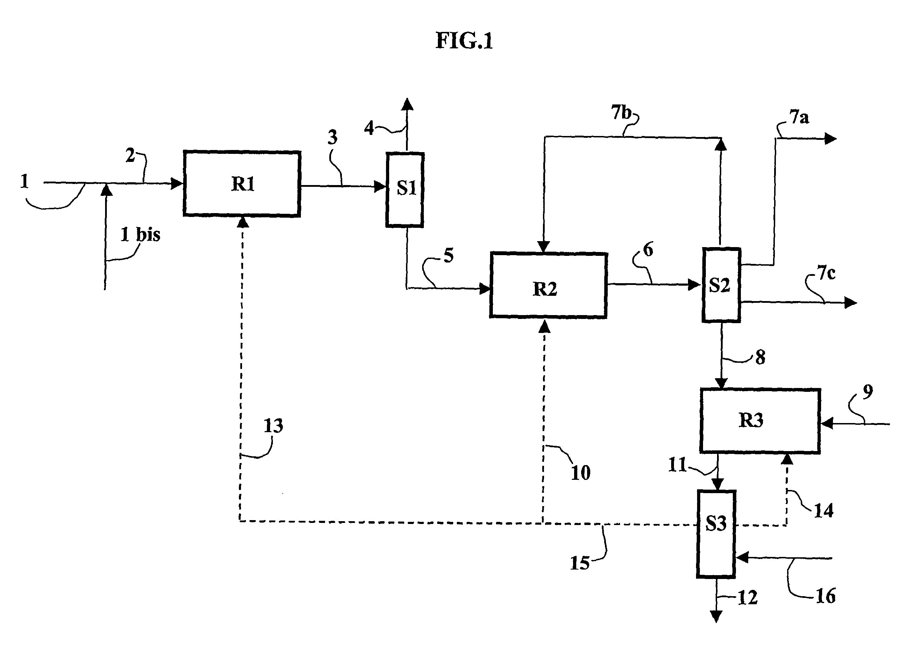

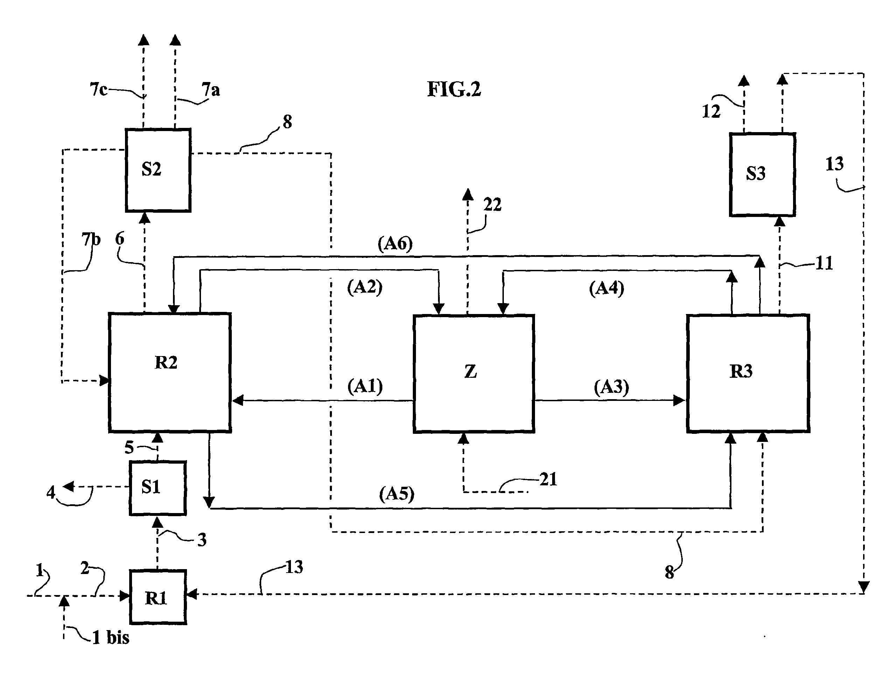

L'invention sera explicitée plus en détail au moyen de la description des figures 1 et 2.

La figure 1 représente une installation pour la mise en oeuvre du procédé selon l'invention dans une première variante à intégration importante entre les étapes du procédé (notamment par des recyclages).

Une charge en C4/C5 provenant d'une unité de vapocraquage (non représentée sur la figure) est introduite par la ligne 1. La ligne 1 bis amène de l'hydrogène ou un gaz riche en hydrogène qui est utilisé pour l'étape d'hydrogénation sélective réalisée en lit fixe dans le réacteur R1 (qui peut comprendre 2 ou 3 zones réactionnelles en série avec refroidissement(s) intermédiaire(s) si nécessaire). La charge et le gaz riche en hydrogène sont introduits dans le réacteur d'hydrogénation R1 par la ligne 2. R1 est également alimenté par un courant de recyclage circulant dans la ligne 13. Le réacteur R1 est ainsi alimenté par deux lignes séparées 2 et 13 sur la figure 1. Il est également possible d'alimenter les charges en mélange par une ligne commune. De même on peut alimenter l'hydrogène à l'intérieur du réacteur et non pas en amont de celui-ci. De telles variantes de réalisation ou moyens techniques équivalents, évidents pour l'homme du métier, sont aussi applicables pour d'autres réacteurs ou zones de séparation représentés sur les figures 1 et 2.
Les effluents du réacteur R1 alimentent par la ligne 3 une zone de fractionnement S1 comprenant une colonne de stabilisation.
On peut éventuellement extraire l'isobutène au niveau de S1 (selon une des techniques exposées ci-après ou d'autres techniques connues par ailleurs), pour réduire la quantité ou éviter la présence d'isobutène dans le réacteur d'oligomérisation. L'isobutène tend en effet à se dimériser en di-isobutène, dont le craquage donne des rendements relativement faibles en propylène, et conduit à un recraquage notable en isobutène, qui tend donc à s'accumuler.
On peut également extraire une fraction au moins des oligomères en C8, pour réduire la quantité ou sensiblement supprimer la fraction riche en di-isobutène soumise au craquage, soit à l'issue de l'oligomérisation, soit après une oligomérisation limitée, comme il a été explicité précédemment.
Les produits légers principalement de l'hydrogène et du méthane, sont évacués par la ligne 4. La coupe C4 hydrogénée sélectivement est introduite par la ligne 5 dans le réacteur d'oligomérisation R2. Une coupe oléfinique recyclée, issue des effluents de l'étape de craquage catalytique, est éventuellement introduite via la ligne 10 dans le réacteur d'oligomérisation. De préférence, cette coupe peut être renvoyée à l'étape d'hydrogénation sélective par la ligne 13 déjà citée, plutôt qu'à l'oligomérisation.

Les effluents de l'oligomérisation sont extraits par la ligne 6 et introduits dans une zone de séparation S2. La zone S2 comprend typiquement un ensemble de colonnes à distiller, simples et/ou réactives et/ou extractives, non représentées sur la figure 1: Après distillation des effluents d'oligomérisation pour récupérer les oligomères, plus lourds, la coupe C4/C5 résiduelle, constituée d'une minorité de composés oléfiniques non transformés et surtout de composés paraffiniques, est évacuée par la ligne 7a. Les oligomères sont en partie au moins transférés par la ligne 8, et introduits dans le réacteur de craquage catalytique R3.

Une autre partie de ces oligomères, ayant donc été séparée des effluents d'oligomérisation (ou d'au moins une étape d'oligomérisation s'il il y en a plusieurs) par prélèvement ou par une ou plusieurs distillations, peut être évacuée par la ligne 7c (et est donc soustraite à la fraction soumise au craquage). Ceci permet de réserver une partie de ces oligomères à d'autres utilisations que la production de propylène, à valorisation éventuellement élevée. La production de propylène est alors moindre, mais la dimension du réacteur de craquage est également réduite. A titre d'exemple, on peut utiliser une partie des oligomères en C10 à C14 comme une des bases pour la fabrication d'alkylbenzènes, linéaires ou non, ou comme bases pour d'autres applications chimiques ou pétrochimiques. On peut également séparer des effluents d'oligomérisation et évacuer une ou plusieurs fractions bouillant dans l'intervalle de distillation de l'essence, du kérosène du gazole, ou du fuel domestique, utilisables comme base(s) pour la fabrication de ces produits. Cette évacuation d'une partie des oligomères, non alimentés au craquage catalytique, est un avantage notable du procédé selon l'invention par rapport aux procédés à une étape unique pour la conversion d'oléfines légères en propylène, qui ne peuvent co-produire des oligomères. Elle peut contribuer également à éliminer indirectement de l'isobutène, lorsque ce composé est présent dans la charge d'oligomérisation, par séparation, par exemple par distillation, et évacuation d'une fraction des oligomères comprenant du di-isobutène et/ou du tri-isobutène (comprenant des dimères ou trimères d'isobutène), par exemple une fraction en C8 ou en C8+. La fraction évacuée peut être séparée par fractionnement des effluents d'oligomérisation, par exemple par distillation. Selon l'invention le fractionnement est considéré au sens large et recouvre également un prélèvement d'une partie des effluents d'oligomérisation ou des oligomères.

Une fraction d'oligomères et/ou de coupe C4 et/ou C5 contenue(s) dans les effluents d'oligomérisation peut éventuellement être recyclée au réacteur d'oligomérisation R2 par la ligne 7b, cette fraction très peu réactive permettant de réduire l'augmentation de température dans le réacteur exothermique R2 (ou les réacteurs en série si l'oligomérisation comprend plusieurs réacteurs). Lorsque l'oligomérisation est réalisée en deux étapes b1) et b3), on peut faire un recyclage spécifique pour chacune des étapes (recyclage d'une partie de l'effluent de la même étape).
Un recyclage d'effluents peut également être mis en oeuvre au niveau de l'étape (ou des étapes ou du réacteur ou des réacteurs) d'hydrogénation sélective, les effluents recyclés pouvant être des effluents d'hydrogénation sélective ou d'oligomérisation, avec éventuellement un recylage global autour de l'ensemble hydrogénation sélective + oligomérisation.

La charge d'oligomères circulant dans la ligne 8 est craquée dans le réacteur de craquage catalytique R3. Le réacteur R3, de préférence un réacteur en lit fixe ou mobile, peut éventuellement être également alimenté par une fraction d'essence introduite par la ligne 9, de façon à augmenter la quantité d'oléfines craquées au moins partiellement en propylène. La fraction en C5 de l'essence, peu réactive au craquage, et éventuellement la fraction en C6, est de préférence alimentée avec la charge en C4/C5, pour être oligomérisée plutôt qu'avec la partie principale de l'essence alimentée à la ligne 9.

La charge globale de l'unité de craquage catalytique comprend donc des oligomères d'oléfines en C4 et/ou C5, et éventuellement de l'essence et/ou une quantité (généralement relativement faible) d'éthylène. Cette charge globale est relativement légère, 50 % poids au moins et généralement 80 % poids et même typiquement 90 % poids au moins (et souvent la totalité) de cette charge bouillant au-dessous de 250°C. Cette charge ne contient typiquement pas de fractions pétrolières telles que du distillat sous vide, et est donc très différente des charges des unités de FCC des raffineries de pétrole.

Les effluents de l'unité de craquage catalytique R3 sont évacués par la ligne 11 et sont introduits dans une zone de séparation S3, qui comprend typiquement un compresseur de gaz et des moyens de distillation.
Lorsque l'installation pour la mise en oeuvre de l'invention est sur un site de vapocraquage, il est très intéressant de pouvoir utiliser le train de fractionnement du vapocraqueur pour le fractionnement des produits. La zone S3 de la figure 1 représente donc une zone de fractionnement commune, d'une part des produits circulant dans la ligne 11, qui sont issus de l'étape de craquage catalytique (du procédé selon l'invention), et d'autre part d'effluents de vapocraquage alimentés par la ligne 16. Les effluents du craquage catalytique représentent une fraction mineure (moins de 50 %, et souvent moins de 30 % molaire des effluents de vapocraquage. En variante, on peut mélanger les effluents de craquage catalytique avec ceux de vapocraquage non pas en amont de la zone de séparation commune, mais après que les effluents de vapocraquage aient subi un fractionnement primaire pour éliminer l'essence (ou au moins l'essence lourde) ou même après le premier étage de compression des effluents gazeux de vapocraquage.
L'invention propose donc également un procédé de conversion associant une étape de vapocraquage et une étape de craquage catalytique, avec de préférence recyclage croisé, dans lequel :
- on soumet à une étape de vapocraquage une charge principale d'hydrocarbures représentant au moins 50 % poids, et généralement au moins 60 % poids de la charge totale,
- on soumet à une étape de craquage catalytique d) telle que précédemment décrite, de préférence en lit fixe ou mobile, une charge secondaire d'hydrocarbures représentant au moins 5 % poids, et généralement au moins 10 % poids, par exemple entre 10 et 40 % poids de la charge totale (constituée par l'addition de la charge principale et de la charge secondaire),
- on refroidit les effluents de vapocraquage et de craquage catalytique,
- on fractionne les effluents de vapocraquage et de craquage catalytique refroidis, au moins en partie dans une zone de fractionnement commune (après éventuellement un fractionnement préliminaire séparé pour éliminer des fractions liquides, la plus grande partie ou sensiblement la totalité des composés gazeux des effluents, notamment ceux comprenant de l'hydrogène et des hydrocarbures comprenant 3 atomes de carbone ou moins, étant de préférence fractionnés dans la zone de fractionnement commune), pour produire au moins de l'éthylène, du propylène, et au moins une coupe oléfinique comprenant des oléfines en C4 et/ou en C5, et de préférence en C4 et en C5,
- on soumet cette coupe oléfinique à au moins une étape d'oligomérisation b) telle que précédemment décrite, pour produire des oligomères,
- on envoie à l'étape d) au moins une partie des oligomères,
- on fractionne de préférence les effluents de l'étape b), dans une étape c), en au moins une fraction riche en oligomères (au moins 50 % poids et souvent au moins 70 % poids, ou même 90 % poids), et au moins une fraction légère comprenant principalement (au moins 50 % poids et souvent au moins 70 % poids, ou même 80 % poids), des hydrocarbures légères ayant 5 atomes de carbone ou moins, par exemple une fraction relativement pauvre en oléfines comprenant principalement des paraffines en C4 et/ou C5,
- on envoie de préférence une partie au moins de la fraction légère de l'étape qui précède à l'étape de vapocraquage.

Les deux dernières étapes précitées sont optionnelles mais préférées selon l'invention: La formation des oligomères permet de séparer aisément par distillation des fractions légères à moins de 8 atomes de carbone (C7-), ou par exemple à moins de 6 atomes de carbone (C5-), comme par exemple une coupe C4/C5. Ces coupes contenant peu d'oléfines , par exemple moins de 20 % poids, du fait de l'oligomérisation de la plus grande partie des oléfines en composés plus lourds, elles constituent de bonnes charges de vapocraquage.
Le procédé de conversion ainsi défini, peut également utiliser une ou plusieurs des variantes du procédé selon l'invention citées précédemment, et par exemple:
- l'utilisation en amont de l'étape b) d'oligomérisation d'une étape a) d'hydrogénation sélective,
- l'utilisation d'une extraction d'isobutène préalablement à l'étape a) ou préalablement à l'étape b), et après l'étape a),
- l'utilisation d'une oligomérisation à au moins deux étapes telle que celle décrite précédemment (étapes b1), b2), b3)),
- l'évacuation à l'étape b2) ou c) d'une partie des oligomères (partie non envoyée à l'étape d)), notamment d'une partie comprenant du di-isobutène et/ou du tri-isobutène.
- l'évacuation (sans recyclage) d'une partie de la coupe C4 des effluents de craquage, pour réaliser une purge d'isobutène.

L'extraction de l'isobutène de coupe(s) recyclée(s) peut être réalisée séparément, ou en même temps qu'une élimination de l'isobutène d'une charge fraîche (externe), par exemple d'une coupe provenant d'un FCC, dans une même installation traitant en mélange les coupes recyclées et la charge fraîche.

L'extraction de l'iso-butène peut être réalisée par distillation extractive, par exemple avec un solvant qui peut être La N-methyl pyrrolidone (NMP) ou le Di-méthyl sulfoxyde (DMSO) ou un isomère de ce dernier.
L'extraction de l'iso-butène, et éventuellement d'autres oléfines branchées, notamment des isoamylènes, peut également comprendre une éthérification de l'isobutène par un alcool, puis une distillation. On peut également réaliser une hydroisomérisation avec distillation réactive, pour séparer l'isobutène du butène (le butène 1 étant transformé en butène 2 séparable de l'isobutène).
On ne sortirait pas du cadre de l'invention en utilisant pour l'extraction d'oléfines branchées (isobutène et/ou isoamylènes) en amont de l'oligomérisation, un ou plusieurs procédés de séparation connus, tels que des extractions liquide-liquide, des éthérifications, ou d'autres procédés tels que des procédés membranaires ou utilisant des adsorbants sélectifs éventuellement à contrecourant simulé.

Les différentes variantes techniques du procédé selon l'invention, décrites pour un procédé combiné de vapocraquage et de craquage catalytique, peuvent également être appliquées lorsque l'installation pour la mise en oeuvre du procédé selon l'invention est associée à un FCC (site de raffinage), ou sur un site mixte (vapocraqueur + FCC), ou sur un site isolé.
On poursuit maintenant la description de la figure 1: La fraction oléfinique débarrassée de l'isobutène et contenant typiquement des paraffines est recyclée au réacteur d'oligomérisation par la ligne 10. De préférence elle est recyclée au réacteur d'hydrogénation sélective R1, par la ligne 15 puis la ligne 13 comme représenté en pointillé sur la figure 1, avant d'être recyclée au réacteur d'oligomérisation R2.
On peut également, après extraction de l'isobutène, réaliser une extraction des paraffines pour recycler ou alimenter à l'hydrogénation sélective ou l'oligomérisation une coupe sans paraffines et sans isobutène, contenant essentiellement des oléfines linéaires.
On peut éventuellement recycler également tout ou de préférence une partie de la coupe essence issue du craquage catalytique de l'étape d), vers le craquage catalytique, comme cela est indiqué par la ligne 14 en pointillé. Les effluents de l'unité de craquage catalytique autres que la coupe C4/C5 recyclée sont évacués par la ligne 12 ainsi que par d'autres lignes non représentées. Une partie ou la totalité de la coupe C4/C5 contenue dans les effluents de craquage peut également être évacuée, et non recyclée.

De façon très générale, la charge traitée à l'oligomérisation peut comprendre une coupe oléfinique C5, seule ou en mélange avec d'autres coupes oléfiniques telles que C4 et/ou C2. On a trouvé que l'on obtenait de meilleurs résultats de craquage (rendement en propylène) en craquant des oligomères formés à partir d'une coupe C5, seule ou co-oligomérisée avec d'autres oléfines, que la charge C5 non oligomérisée. L'amélioration concerne à la fois la sélectivité de craquage vers le propylène et la conversion en simple passe des oligomères, qui est accrue. On pense que cette accroissement observé de la conversion en simple passe, pour des oligomères par rapport aux oléfines de départ, provient notamment de l'augmentation du poids moléculaire, et est notable pour toutes les charges oléfiniques prévues dans la présente demande.

Selon une autre variante, on peut recycler la coupe C4/C5 sans extraction de l'isobutène. La charge C4/C5 brute, après hydrogénation sélective, est alors oligomérisée dans R2 et séparée dans S2. S2 peut comprendre alors seulement une séparation des oligomères (par distillation), envoyés au réacteur R3 par la ligne 8, la coupe C4/C5 résiduelle (contenue dans les effluents d'oligomérisation), essentiellement paraffinique, étant évacuée par la ligne 7a. Il est alors préférable de prévoir une évacuation (sans recyclage) d'une partie de la coupe C4 issue du craquage, et/ou de ne pas alimenter à l'étape de craquage une partie des oligomères, afin de réaliser une purge directe ou indirecte d'isobutène. Cette évacuation d'oligomères, notamment d'oligomères comprenant du di-isobutène et/ou du tri-isobutène peut également être faite dans le cadre d'une oligomérisation en une ou deux étapes, comme cela a déjà été explicité.
On peut également recycler des fractions oléfiniques en C6.

La Figure 2 décrit une installation pour la mise en oeuvre d'une variante du procédé selon l'invention utilisant un même catalyseur circulant en lit fluidisé dans et entre trois zones distinctes : le réacteur d'oligomérisation R2, le réacteur de craquage R3, et une zone Z de régénération commune du catalyseur. Les flux solides sont représentés en traits pleins et les flux de charge, d'effluents ou des différents recyclages sont représentés en traits pointillés.

Le catalyseur est régénéré dans la zone Z à des températures élevées de l'ordre de 500°C à 750°C, des niveaux de pression compris typiquement entre 0,1 MPa et 4 MPa et préférentiellement compris entre 0,1 et 3 MPa, à l'aide d'un gaz de combustion, qui est généralement de l'air ou de l'air dilué par de l'azote et/ou un recyclage de gaz de combustion.

L'air (ou air dilué) alimente la zone Z par la ligne 21 et les fumées de combustion sont évacuées par la ligne 22.

Dans la zone Z, la régénération du catalyseur peut être effectuée en une ou plusieurs étapes à des températures et des pressions partielles en oxygène différentes. Il est ainsi possible de brûler le coke déposé sur le catalyseur d'abord avec une pression partielle d'oxygène faible, puis à plus hautes températures et pression partielle d'oxygène, sans risque d'emballement de la réaction de combustion, en découplant le processus de régénération en deux étapes. La zone Z étant sous atmosphère d'oxygène, il est souhaitable d'avoir des zones tampons en amont et en aval de la dite zone avec des flux inertes, par exemple de l'azote, de manière à prévenir les éventuelles fuites d'oxygène par les lignes de transfert de catalyseur entre les réacteurs R2, R3 et la zone Z. De façon plus générale l'installation peut comprendre des moyens de génie chimique non représentés, tels par exemple que des trémies de catalyseur, des moyens de mélange d'un flux de catalyseur avec un liquide et/ou un gaz, des sas de mélange, de pressurisation ou de dépressurisation, de mouillage, ou d'inertage, des moyens de stripage, de réchauffage ou de refroidissement du catalyseur, par exemple un stripeur de solide fluidisé ou un échangeur thermique de réchauffage ou de refroidissement de solide fluidisé etc....

Un flux de catalyseur régénéré est typiquement convoyé de la zone Z vers le réacteur d'oligomérisation R2 par la ligne de transfert A1, et retourne partiellement désactivé de R2 à la zone Z par la ligne A2.

De même, un autre flux de catalyseur régénéré est typiquement convoyé de la zone Z vers le réacteur de craquage catalytique R3 par la ligne de transfert A3, et retourne partiellement désactivé de R3 à la zone Z par la ligne A4.

L'installation peut également fonctionner avec un mode différent: La totalité du catalyseur régénéré peut être convoyée vers le réacteur R3 par la ligne A3, puis transféré du réacteur R3 vers le réacteur R2 par la ligne A6, puis retourner à la zone Z par la ligne A2.

De façon similaire, la totalité du catalyseur régénéré peut être convoyée vers le réacteur R2 par la ligne A1, puis transféré du réacteur R2 vers le réacteur R3 par la ligne A5, puis retourner à la zone Z par la ligne A4.

L'installation peut aussi fonctionner en combinant ces différents modes de circulation qui mettent alors en oeuvre des flux de catalyseur partiels (et non pas la totalité du flux de catalyseur régénéré).
La réalisation de températures plus basses dans le réacteur R2 d'oligomérisation (par comparaison avec le réacteur R3 de craquage) peut être obtenue en refroidissant (ou refroidissant plus) le flux de catalyseur alimentant R2 et/ou en alimentant R2 par des réactifs à relativement plus basse température. Ces moyens de refroidissement ne sont pas représentés sur la figure.

Les pressions opératoires peuvent être voisines ou sensiblement identiques dans les trois zones : réacteur R2, réacteur R3, et régénération Z pour faciliter la circulation du catalyseur, mais on peut également fonctionner à des pressions différentes, par exemple à une pression supérieure pour l'oligomérisation.

Les autres éléments référencés de la figure 2 ont déjà été décrits pour la figure 1.
La figure 2 peut également représenter une installation pour la mise en oeuvre du procédé selon l'invention avec R2 et R3 fonctionnant en lit mobile, et non en lit fluidisé (avec circulation continue ou semi-continue (par intermittence) d'un catalyseur sous forme de grains, par exemple des billes de diamètre compris entre 1 et 3 mm).

Le procédé selon l'invention n'est pas limité aux éléments précédemment décrits, et peut être mis en oeuvre selon des variantes ou avec des modes de réalisation non décrits dans la présente description mais déjà connus de l'homme du métier.

### Exemple 1 (selon l'invention) :

L'exemple suivant sur unité pilote illustre l'invention sans en limiter la portée.
On alimente à l'oligomérisation une coupe C4 de vapocraquage additionnée d'une coupe C4/C5 recyclée de l'étape de craquage. Cette coupe est préalablement hydrogénée sélectivement et débarrassée de l'isobutène qu'elle contenait initialement.
Les compositions en pourcentage massique de la charge et de l'effluent d'oligomérisation sont données au tableau 1.
Les conditions opératoires de la zone d'oligomérisation sont les suivantes : pression : 5,5 MPa, température : 220 °C ; VVH =1 h-1. Le catalyseur utilisé est une zéolithe de type MFI avec un rapport Si/Al de 48. Il est mis en oeuvre sous forme de billes de diamètre moyen 2 mm.
Les oligomères produits, qui contiennent principalement des oligomères oléfiniques en C8 et, en quantités plus faibles en C12, alimentent la zone de craquage qui opère avec les conditions opératoires suivantes :
- pression : 0,2 MPa,
- température : 520°C,
- VVH = 10 h-1.
Le catalyseur utilisé contient 30 %poids de zéolithe ZSM-5, dispersée dans une matrice de silice-alumine. Il est mis en oeuvre sous forme de billes de 2 mm de diamètre moyen.
Les rendements du craquage des oligomères sont donnés dans le tableau 2.
La fraction C4/C5 de craquage (hors isobutène) est alors recyclée, comme il a déjà été mentionné.
Le calcul de rendement global en propylène, en tenant compte du bilan coke (environ 2 % à l'étape de craquage) conduit à un rendement de 48 % par rapport aux oléfines de la charge initiale. Ce rendement pourrait être légèrement augmenté en recyclant les oléfines en C4/C5 non converties en sortie de l'oligomérisation.
En variante, si l'on souhaite réaliser une production conjointe de propylène et d'essence (et éventuellement de kérosène), on peut augmenter le débit de la section d'oligomérisation (sans modifier le craquage) et extraire une coupe additionnelle d'oligomères constituant une base d'essence et éventuellement de kérosène.

**Tableau 1**

| % massique | Entrée oligo | Sortie oligo |
|---|---|---|
| Paraffines C4/C5 | 11,99 | 15,65 |
| Isobutène | 0,0 | 0,0 |
| Oléfines C4/C5(hors isobutène) | 88,01 | 0,83 |
| Oligomères | 0,0 | 83,52 |
| Total | 100,0 | 100,0 |

**Tableau 2**

| % massique | Sortie craquage |
|---|---|
| H2 | 0,41 |
| CH4 | 1,22 |
| C2H4 | 4,59 |
| C2H6 | 2,24 |
| C3H6 | 40,79 |
| C3H8 | 3,57 |
| Paraffines C4/C5 | 6,01 |
| isobutène | 11,83 |
| Oléfines C4/C5 hors isobutène | 25,26 |
| Essence | 4,08 |
| Total | 100,0 |

### Exemple 2: Oligomérisation de diverses charges oléfiniques:

On dispose de deux coupes oléfiniques C4 et C5 (qui peuvent par exemple provenir d'un FCC), pour lesquelles on réalise une oligomérisation préalable: soit de la coupe C4 seule, soit de la coupe C4 en mélange avec la coupe C5 (co-oligomérisation). Les effluents de ces oligomérisations seront utilisés dans les exemples 3 et 4 qui suivent.
Les conditions d'oligomérisation sont identiques à celles de l'exemple 1.
La composition des charges et des effluents d'oligomérisation est donnée dans le tableau 3 suivant :

**Tableau 3**

| Charge ou Effluent d'oligomérisation kg/h | Coupe C4 (charge) | Coupe C5 (charge) | Effluent d'Oligomérisation de Coupe C4 | Effluent d' Oligomérisation de Coupe (C4 + C5) |
|---|---|---|---|---|
| Paraffines C4 | 7649 | 0 | 7871 | 7871 |
| Isobutène | 125 | 0 | 6 | 6 |
| Butène 1 | 2025 | 0 | 103 | 106 |
| Butène 2 | 5069 | 0 | 254 | 324 |
| Paraffines C5 | 0 | 7598 | 0 | 7844 |
| Oléfines C5 | 0 | 9838 | 0 | 2103 |
| Coupe C6⁺ (1) | 0 | 0 | 6633 | 14048 |
| Total | 14867 | 17435 | 14867 | 32302 |

| | | | | |
|---|---|---|---|---|
| (1) Une coupe Cn⁺ comprend, par définition les fractions ayant au moins n atomes de carbone. | | | | |

### Exemple 3 selon l'invention:

On craque catalytiquement, dans les mêmes conditions que celles de l'exemple 1, la coupe C5⁺ issue de l'oligomérisation (co-oligomérisation) de la coupe (C4 + C5) selon l'exemple 2 (dernière colonne du tableau 3). Les rendements de craquage, exprimés comme dans le cas de l'exemple 1, en % pds par rapport à la fraction oléfinique de la charge C4 + C5, hors isobutène, sont donnés dans le tableau 4 suivant:

**Tableau 4**

| Rendements %pds | Sortie craquage |
|---|---|
| H2 | 0,39 |
| CH4 | 1,15 |
| C2H4 | 5,20 |
| C2H6 | 2,44 |
| C3H6 | 28,73 |
| C3H8 | 3,35 |
| Paraffines C4/C5 | 5,65 |
| Isobutène | 11,11 |
| Oléfines C4/C5 hors isobutène | 37,40 |
| Essence | 4,60 |
| Total | 100,00 |

Si le réacteur de craquage, par exemple dans une unité FCC ou une unité en lit fixe ou mobile, ou le compresseur de gaz craqués, ou l'unité de traitement des gaz sont limités en capacité, on peut également alimenter au craquage non pas la coupe C5⁺ issue de l'oligomérisation, mais la coupe C6⁺ issue de l'oligomérisation, pour réduire le débit d'oligomères craqués, avec une perte de propylène relativement faible.
Si l'on veut encore réduire la charge d'oligomères envoyés au craquage, on peut n'envoyer vers le craquage que la fraction C8⁺ ou même C9⁺. Ces diverses variantes sont également utilisables dans le cas ou l'on fait une oligomérisation en 2 étapes b1) et b3). On peut par exemple réaliser une première oligomérisation b1) d'une coupe C4/C5, évacuer des oligomères C8⁺ vers une zone de fractionnement pour la préparation de bases d'essence et/ou de kérosène, alimenter la fraction C4/C5 résiduelle vers une seconde oligomérisation b3), puis séparer des seconds oligomères C8⁺ ou C9⁺ que l'on alimente au craquage.

### Exemple 4 (comparatif):

On craque catalytiquement, dans les conditions de l'exemple 1, la coupe C5 initiale (donc sans oligomérisation préalable), additionnée de la fraction C5⁺ comprise dans les effluents de l'oligomérisation de la coupe C4 selon l'exemple 2. Dans cet exemple comparatif, seule la coupe C4 est oligomérisée (au fins typiquement de l'élimination des butanes après cette oligomérisation et avant le craquage). La coupe C5, qui contient relativement moins de paraffines que la coupe C4, est craquée directement, en mélange avec les oligomères C5⁺ obtenus par oligomérisation de la coupe C4. Les rendements de craquage sont donnés dans le tableau suivant:

**Tableau 5**

| Rendements Kg/h | Sortie craquage |
|---|---|
| H2 | 0,24 |
| CH4 | 0,73 |
| C2H4 | 4,87 |
| C2H6 | 2,13 |
| C3H6 | 18,17 |
| C3H8 | 2,12 |
| Paraffines C4/C5 | 3,57 |
| Isobutène | 7,03 |
| Oléfines C4/C5 hors isobutène | 59,03 |
| Essence | 2,11 |
| Total | 100,00 |

On peut constater que le rendement en propylène de cet exemple comparatif est notablement plus bas que celui de l'exemple 3 selon l'invention. Ceci montre l'intérêt de co-oligomériser des coupes complexes comprenant des oléfines ayant des nombres d'atomes de carbone différents, avant de réaliser le craquage, et ceci pour améliorer la sélectivité vers le propylène et la conversion en simple passe (Note: les exemples 3 et 4 ne peuvent être comparés à l'exemple 1, car, outre la charge qui est différente, ils correspondent à un craquage en simple passe, sans recyclage des oléfines reformées au cours du craquage).

Le procédé selon l'invention permet donc, selon différentes variantes, d'améliorer la production de propylène pour une charge donnée. Il permet également de réduire les recyclages grâce à une conversion plus élevée par passe, et typiquement d'améliorer la sélectivité de craquage vers le propylène. Il peut permettre également de co-produire de l'essence d'indice d'octane élevé (notamment de l'essence riche en di-isobutène) et du propylène, ou bien du di-isobutène (mélange des deux isomères) et du propylène, ou encore des bases d'essence et / ou de kérosène et / ou de gazole, et du propylène.

## Revendications

1. Procédé de conversion catalytique d'une charge hydrocarbonée comprenant des oléfines à 4 atomes de carbone, ledit procédé étant **caractérisé par** l'enchaînement des étapes suivantes :
- au moins une étape d'oligomérisation dans laquelle on réalise dans au moins un réacteur une oligomérisation catalytique desdites oléfines à 4 atomes de carbone en présence d'autres oléfines contenues également dans la charge, ces dites autres oléfines appartenant au groupe constitué par l'éthylène d'une part, et les oléfines à 5 ou 6 atomes de carbone d'autre part, pour obtenir des oligomères, puis,
- une étape de craquage catalytique d'une partie au moins desdits oligomères, dans un réacteur différent du réacteur d'oligomérisation, pour produire notamment du propylène.

2. Procédé selon la revendication 1, dans lequel la charge d'oligomérisation comprend au moins 10% poids d'oléfines en C4 et au moins 10% poids d'oléfines en C5, avec un rapport massique [R2 = oléfines en C5 / oléfines en C4] tel que 0,3 < R2 < 3.

3. Procédé selon l'une des revendications 1 et 2 de conversion catalytique d'une charge hydrocarbonée comprenant des oléfines à 4 atomes de carbone, et comprenant en particulier de l'isobutène, ledit procédé étant **caractérisé par** l'enchaînement des étapes suivantes :
- une première étape d'oligomérisation limitée,
- une étape de fractionnement des effluents de ladite première étape d'oligomérisation limitée, pour produire au moins une coupe qui est évacuée directement sans alimenter lesdites étapes subséquentes d'oligomérisation finale et de craquage catalytique, cette coupe évacuée comprenant du di-isobutène et/ou du tri-isobutène,
- une étape d'oligomérisation finale de l'effluent de ladite étape de fractionnement ou au moins de fractions oléfiniques en C4 et/ou C5 comprises dans cet effluent, après évacuation de la coupe précitée comprenant du di-isobutène et/ou du tri-isobutène,
- ladite étape de craquage catalytique.

4. Procédé selon la revendication 3, dans lequel on réalise:
- l'étape d'oligomérisation limitée avec une charge constituée par une coupe C4 seule,
- l'étape d'oligomérisation finale en rajoutant une coupe C5 ou C2 + C5 aux butènes non convertis dans l'étape d'oligomérisation limitée.

5. Procédé de conversion catalytique d'une charge hydrocarbonée comprenant une quantité substantielle d' oléfines à 5 atomes de carbone, ledit procédé étant **caractérisé par** l'enchaînement des étapes suivantes :
- au moins une étape d'oligomérisation dans laquelle on réalise dans au moins un réacteur une oligomérisation catalytique des oléfines contenues dans la charge, puis,
- une étape de craquage catalytique d'une partie au moins des oligomères produits, dans un réacteur différent du réacteur d'oligomérisation, pour produire notamment du propylène.

6. Procédé selon l'une des revendications 1 à 5, dans lequel la charge du réacteur d'oligomérisation comprend de 0,5 à 15 % poids d'éthylène.

7. Procédé selon l'une des revendications 1 à 6, dans lequel la charge du réacteur d'oligomérisation comprend de 0,5 à 15 % poids d'éthylène par rapport à la somme des oléfines en C4, C5, et C6.

8. Procédé selon l'une des revendications précédentes, dans lequel la charge du réacteur d'oligomérisation comprend au moins 50% poids d'hydrocarbures en C4+ C5+ C6, au moins 10 % poids d'oléfines à 4 atomes de carbone, ainsi que des oléfines à 5 et/ou 6 atomes de carbone, avec un rapport massique [R1 = (oléfines en C5 + oléfines en C6) / oléfines en C4] supérieur à 0,15.

9. Procédé selon l'une des revendications précédentes , dans lequel la charge du réacteur d'oligomérisation comprend au moins 50% poids d'hydrocarbures en C4+ C5+ C6, au moins 10 % poids d'oléfines à 4 atomes de carbone, ainsi que des oléfines à 5 atomes de carbone, avec un rapport massique [R2 = oléfines en C5 / oléfines en C4] supérieur à 0,15.

10. Procédé selon l'une des revendications précédentes, dans lequel la charge du réacteur d'oligomérisation contient des composés dioléfiniques et/ou acétyléniques, et dans lequel cette charge est soumise préalablement à une étape a) d'hydrogénation sélective pour sensiblement éliminer ces composés dioléfiniques et/ou acétyléniques.

11. Procédé selon l'une des revendications précédentes, dans lequel le catalyseur utilisé pour l'étape d'oligomérisation comprend un solide acide présentant une sélectivité de forme, ce catalyseur comprenant au moins une zéolithe, cette zéolithe comprenant du silicium et au moins un élément choisi dans le groupe formé par l'aluminium, le fer, le gallium, le phosphore, le bore, et de préférence l'aluminium, la zéolithe présentant une sélectivité de forme utilisée étant du groupe constitué par les zéolithes de l'un des types structuraux suivants : MEL, MFI, NES, EUO, FER, CHA, MFS, MWW, ou du groupe des zéolithes suivantes: NU-85, NU-86, NU-88 et IM-5.

12. Procédé selon l'une des revendications précédentes, dans lequel le catalyseur de l'étape d) de craquage catalytique comprend une zéolithe, le craquage étant réalisé à une température comprise entre 450°C et 650°C, une pression comprise entre 0,1 et 0,5 MPa.

13. Procédé selon l'une des revendications précédentes, dans lequel le catalyseur utilisé pour l'étape de craquage comprend une zéolithe présentant une sélectivité de forme de type structural MFI, seule ou en mélange avec une autre zéolithe présentant une sélectivité de forme choisie dans le groupe formé par les zéolithes de type structural de l'un des types suivants : MEL (par exemple la ZSM-11), NES, EUO, FER, CHA (par exemple la SAPO-34), MFS, MWW, ou le groupe des zéolithes suivantes : NU-85, NU-86, NU-88 et IM-5, et dans lequel la ou les zéolithe(s) utilisée(s) présentant une sélectivité de forme présentent un rapport Si/Al supérieur à 12.

14. Procédé selon l'une des revendications précédentes, dans lequel le catalyseur utilisé pour l'étape de craquage comprend une ou plusieurs zéolithe(s) présentant une sélectivité de forme, la proportion de zéolithe(s) présentant une sélectivité de forme étant comprise entre 70 et 100 % poids par rapport à la quantité totale de zéolithe(s).

15. Procédé selon la revendication 11 et la revendication 13, dans lequel le catalyseur utilisé à l'étape de craquage comprend une zéolithe présentant une sélectivité de forme de rapport Si/Al différent de la ou des zéolithes présentant une sélectivité de forme comprise(s) dans le catalyseur utilisé à l'étape d'oligomérisation.

16. Procédé selon l'une quelconque des revendications précédentes, dans lequel le réacteur de craquage catalytique est un réacteur en lit fixe ou mobile ou fluidisé.

## Claims

1. Process for catalytic conversion of a hydrocarbon charge comprising olefins with 4 carbon atoms, said process being **characterized by** the following sequence of stages:
- at least one stage of oligomerization in which a catalytic oligomerization of said olefins with 4 carbon atoms is carried out in at least one reactor in the presence of other olefins also contained in the charge, said other olefins belonging to the group comprising ethylene on the one hand, and olefins with 5 or 6 carbon atoms on the other hand, to obtain oligomers, then,
- a stage for catalytic cracking a proportion at least of said oligomers in a reactor different from the oligomerization reactor, in particular to produce propylene.

2. Process according to claim 1, in which the oligomerization charge comprises at least 10 wt.% of olefins with 4 carbon atoms and at least 10 wt.% of olefins with 5 carbon atoms, with a mass ratio [R2 = C5 olefins / C4 olefins] such as 0.3 < R2 < 3.

3. Process according to one of claims 1 and 2, for catalytic conversion of a hydrocarbon charge comprising olefins with 4 carbon atoms and comprising in particular isobutene, said process being **characterized by** the following sequence of stages:
- a first stage of limited oligomerization,
- a stage of fractionation of the effluents of said first stage, to produce at least one cut which is evacuated directly without feeding the subsequent stages for final oligomerization and for catalytic cracking, said evacuated cut comprising di-isobutene and/or tri-isobutene,
- a stage of final oligomerization of the effluent of said fractionation stage or at least of C4 and/or C5 olefinic fractions contained in said effluent, after evacuation of the aforementioned cut comprising di-isobutene and/or tri-isobutene,
- said catalytic cracking stage.

4. Process according to claim 3, in which the following are carried out:
- the first stage of limited oligomerization with a charge consisting of a C4 cut alone,
- the final oligomerization stage, adding a C5 or C2 + C5 cut to the butenes that were not converted in the first stage of limited oligomerization.

5. Process for catalytic conversion of a hydrocarbon charge comprising a substantial quantity of olefins with 5 carbon atoms, said process being **characterized by** the following sequence of stages:
- at least one stage of oligomerization in which a catalytic oligomerization of the olefins contained in the charge is carried out in at least one reactor, then,
- a stage for catalytic cracking a proportion at least of the oligomers produced in a reactor different from the oligomerization reactor, in particular to produce propylene.

6. Process according to one of claims 1 to 5, in which the charge of the oligomerization reactor comprises from 0.5 to 15 wt.% of ethylene.

7. Process according to one of claims 1 to 6, in which the charge of the oligomerization reactor comprises from 0.5 to 15 wt.% of ethylene relative to the total of the C4, C5 and C6 olefins.

8. Process according to one of the preceding claims, in which the charge of the oligomerization reactor comprises at least 50 wt.% of C4+C5+C6 hydrocarbons, at least 10 wt.% of olefins with 4 carbon atoms, as well as olefins with 5 and/or 6 carbon atoms, with a mass ratio [R1 = (C5 olefins + C6 olefins) / C4 olefins] greater than 0.15.

9. Process according to one of the preceding claims, in which the charge of the oligomerization reactor comprises at least 50 wt.% of C4+C5+C6 hydrocarbons, at least 10 wt.% of olefins with 4 carbon atoms, as well as olefins with 5 carbon atoms, with a mass ratio [R2 = C5 olefins / C4 olefins] greater than 0.15.

10. Process according to one of the preceding claims, in which the charge of the oligomerization reactor comprises diolefinic and/or acetylenic compounds, and in which said charge is first subjected to a stage a) of selective hydrogenation for practically eliminating these diolefinic and/or acetylenic compounds.

11. Process according to one of the preceding claims, in which the catalyst used for the oligomerization stage comprises an acidic solid possessing shape selectivity, said catalyst comprising at least one zeolite, said zeolite comprising silicon and at least one element chosen from the group formed by aluminium, iron, gallium, phosphorus, boron, and preferably aluminium, the zeolite exhibiting shape selectivity used being from the group comprising the zeolites of one of the following structural types: MEL, MFI, NES, EUO, FER, CHA, MFS, MWW, or from the group of the following zeolites: NU-85, NU-86, NU-88 and IM-5.

12. Process according to one of the preceding claims, in which the catalyst of Stage d) of catalytic cracking comprises a zeolite, cracking being carried out at a temperature comprised between 450°C and 650°C, and a pressure comprised between 0.1 and 0.5 MPa.

13. Process according to one of the preceding claims, in which the catalyst used for the cracking stage comprises a zeolite exhibiting shape selectivity ofju structural type MFI, alone or mixed with another zeolite exhibiting shape selectivity chosen from the group comprising the zeolites of one of the following structural types: MEL (for example ZSM-11), NES, EUO, FER, CHA (for example SAPO-34), MFS, MWW, or the group of the following zeolites: NU-85, NU-86, NU-88 and IM-5, and in which the zeolite or zeolites used exhibiting shape selectivity have an Si/Al ratio greater than 12.

14. Process according to one of the preceding claims, in which the catalyst used for the cracking stage comprises one or more zeolites exhibiting shape selectivity, the proportion of zeolite(s) exhibiting shape selectivity being comprised between 70 and 100 wt.% relative to the total quantity of zeolite(s).

15. Process according to claim 11 and claim 13, in which the catalyst used in the cracking stage comprises a zeolite exhibiting shape selectivity with Si/Al ratio different from the zeolite or zeolites exhibiting shape selectivity contained in the catalyst used in the oligomerization stage.

16. Process according to any one of the preceding claims, in which the catalytic cracking reactor is a fixed-bed or moving-bed or fluidized-bed reactor.

## Patentansprüche

1. Verfahren zur katalytischen Umwandlung einer Kohlenwasserstoffbeschickung, die Olefine mit 4 Kohlenstoffatomen umfasst, wobei das Verfahren durch die Abfolge der folgenden Schritte **gekennzeichnet** ist:
- mindestens ein Schritt der Oligomerisation, wobei in mindestens einem Reaktor eine katalytische Oligomerisation der Olefine mit 4 Kohlenstoffatomen realisiert wird, in Gegenwart anderer Olefine, die ebenfalls in der Beschickung enthalten sind, wobei diese anderen Olefine einerseits zur Gruppe gehören, die aus Ethylen einerseits und aus den Olefinen mit 5 oder 6 Kohlenstoffatomen andererseits besteht, um Oligomere zu erhalten, dann,
- ein Schritt des katalytischen Crackens mindestens eines Teils der Oligomere, in einem Reaktor, der nicht der Oligomerisationsreaktor ist, um vor allem Propylen zu produzieren.

2. Verfahren nach Anspruch 1, wobei die Oligomerisationsbeschickung mindestens 10 Gew.-% C4-Olefine und mindestens 10 Gew.-% C5-Olefine, mit einem Massenverhältnis [R2 = C5-Olefine/C4-Olefine], von etwa 0,3 < R2 < 3, umfasst.

3. Verfahren nach einem der Ansprüche 1 und 2 zur katalytischen Umwandlung einer Kohlenwasserstoffbeschickung, die Olefine mit 4 Kohlenstoffatomen umfasst, und die insbesondere Isobuten umfasst, wobei das Verfahren durch die Abfolge der folgenden Schritte **gekennzeichnet** ist:
- ein erster Schritt der begrenzten Oligomerisation,
- ein Schritt der Fraktionierung der Abflüsse aus dem ersten Schritt der begrenzten Oligomerisation, um mindestens einen Schnitt zu produzieren, der direkt abgeleitet wird ohne die nachfolgenden Schritte der finalen Oligomerisation und des katalytischen Crackens zu versorgen, wobei dieser abgeleitete Schnitt, Diisobuten und/oder Triisobuten umfasst,
- ein Schritt der finalen Oligomerisation des Abflusses aus dem Schritt der Fraktionierung oder mindestens der C4-und/oder C5-Olefinfraktionen, die dieser Abfluss umfasst, nach dem Ableiten des vorgenannten Schnitts, der Diisobuten und/oder Triisobuten umfasst,
- den Schritt des katalytischen Crackens.

4. Verfahren nach Anspruch 3, wobei realisiert wird:
- der Schritt der begrenzten Oligomerisation mit einer Beschickung, die nur aus einem C4-Schnitt besteht,
- der Schritt der finalen Oligomerisation, indem den im Schritt der beschränkten Oligomerisation nicht umgewandelten Butenen ein C5- oder C2+ C5-Schnitt zugefügt wird.

5. Verfahren zur katalytischen Umwandlung einer Kohlenwasserstoffbeschickung, die eine beträchtliche Menge an Olefinen mit 5 Kohlenstoffatomen umfasst, wobei das Verfahren durch die Abfolge der folgenden Schritte **gekennzeichnet** ist:
- mindestens ein Schritt der Oligomerisation, wobei in mindestens einem Reaktor eine katalytische Oligomerisation der Olefine realisiert wird, die in der Beschickung enthalten sind, dann,
- ein Schritt des katalytischen Crackens mindestens eines Teils der produzierten Oligomere, in einem Reaktor, der nicht der Reaktor der Oligomerisation ist, um vor allem Propylen zu produzieren.

6. Verfahren nach einem der Ansprüche 1 bis 5, wobei die Beschickung des Oligomerisationsreaktors zwischen 0,5 und 15 Gew.-% Ethylen umfasst.

7. Verfahren nach einem der Ansprüche 1 bis 6, wobei die Beschickung des Oligomerisationsreaktors zwischen 0,5 und 15 Gew.-% Ethylen, bezogen auf die Summe der C4-, C5- und C6-Olefine umfasst.

8. Verfahren nach einem der vorhergehenden Ansprüche, wobei die Beschickung des Oligomerisationsreaktors mindestens 50 Gew.-% C4+C5+C6-Kohlenwasserstoffe, mindestens 10 Gew.-% Olefine mit 4 Kohlenstoffatomen, sowie Olefine mit 5 und/oder 6 Kohlenstoffatomen, mit einem Massenverhältnis [R1 = (C5-Olefine + C6-Olefine)/C4-Olefine] von über 0,15, umfasst.

9. Verfahren nach einem der vorhergehenden Ansprüche, wobei die Beschickung des Oligomerisationsreaktors mindestens 50 Gew.-% C4+C5+C6-Kohlenwasserstoffe, mindestens 10 Gew.-% Olefine mit 4 Kohlenstoffatomen, sowie Olefine mit 5 Kohlenstoffatomen mit einem Massenverhältnis [R2 = C5-Olefine/C4-Olefine] von über 0,15, umfasst.

10. Verfahren nach einem der vorhergehenden Ansprüche, wobei die Beschickung des Oligomerisationsreaktors diolefinische und/oder acetylenische Verbindungen enthält, und wobei diese Beschickung vorher einem Schritt a) der selektiven Hydrierung unterzogen wird, um diese diolefinischen und/oder acetylenischen Verbindungen im Wesentlichen zu entfernen.

11. Verfahren nach einem der vorhergehenden Ansprüche, wobei der Katalysator, der für den Schritt der Oligomerisation verwendet wird, eine feste Säure umfasst, die eine Formselektivität aufweist, wobei dieser Katalysator mindestens einen Zeolithen umfasst, wobei dieser Zeolith Silicium und mindestens ein Element umfasst, ausgewählt aus der Gruppe, gebildet aus Aluminium, Eisen, Gallium, Phosphor, Bor und bevorzugt Aluminium, wobei der verwendete Zeolith, der eine Formselektivität aufweist, aus der Gruppe stammt, die aus den Zeolithen mit einer der folgenden Strukturtypen besteht: MEL, MFI, NES, EUO, FER, CHA, MFS, MWW oder aus der Gruppe der folgenden Zeolithe: NU-85, NU-86, NU-88 und IM-5.

12. Verfahren nach einem der vorhergehenden Ansprüche, wobei der Katalysator aus Schritt d) zum katalytischen Cracken einen Zeolithen umfasst, wobei das Cracken bei einer Temperatur im Bereich zwischen 450 °C und 650 °C, einem Druck im Bereich zwischen 0,1 und 0,5 MPa realisiert wird.

13. Verfahren nach einem der vorhergehenden Ansprüche, wobei der Katalysator, der für den Schritt des Crackens verwendet wird, einen Zeolithen umfasst, der eine Formselektivität von Strukturtyp MFI aufweist, allein oder als Mischung mit einem anderen Zeolithen, der eine Formselektivität aufweist, ausgewählt aus der Gruppe, gebildet aus den Zeolithen mit dem Strukturtyp einer der folgenden Typen: MEL (zum Beispiel ZSM-11), NES, EUO, FER, CHA (zum Beispiel SAPO-34), MFS, MWW oder der folgenden Gruppe von Zeolithen: NU-85, NU-86, NU-88 und IM-5, und wobei der oder die verwendete(n) Zeolith(en) eine Formselektivität aufweist (aufweisen), die ein Si/Al-Verhältnis von mehr als 12 aufweist.

14. Verfahren nach einem der vorhergehenden Ansprüche, wobei der für den Schritt des Crackens verwendete Katalysator einen oder mehrere Zeolithen umfasst, der (die) eine Formselektivität aufweist (aufweisen), wobei der Anteil an Zeolith(en), der (die) eine Formselektivität aufweist (aufweisen), im Bereich zwischen 70 und 100 Gew.-%, bezogen auf die Gesamtmenge an Zeolith(en) liegt.

15. Verfahren nach Anspruch 11 und Anspruch 13, wobei der für den schritt des Crackens verwendete Katalysator einen Zeolithen umfasst, der eine Formselektivität aufweist, mit einem Si/Al-Verhältnis, das sich von dem des oder den Zeolithen unterscheidet, der (die) eine Formselektivität aufweist (aufweisen), den der Katalysator umfasst, der im Schritt der Oligomerisation verwendet wird.

16. Verfahren nach einem der vorhergehenden Ansprüche, wobei der Reaktor zum katalytischen Cracken ein Festbett, Bewegtbett- oder Fliessbettreaktor ist.
